# EUROPEAN PATENT APPLICATION

(11) **EP 4 053 557 A1**
(43) Date of publication of application: **07.09.2022**
(21) Application number: 22166448.5
(22) Date of filing: 24.11.2017
(51) Int. Cl.: G01N 33/574, C12Q 1/68

(54) **IL-29 AS A MARKER FOR A PANCREATIC DISEASE**

(30) Priority: 24.11.2016 KR 20160157502
(62) Divisional of application: 17872958.8
(71) Applicant: Huvet Bio, Inc., Seoul 05836 (KR)
(72) Inventor: LEE, Dong Ki, 13556 Seongnam-si Gyeonggi-do (KR); HAAM, Seung Joo, 04427 Seoul (KR); YOOK, Jong In, 06004 Seoul (KR); LEE, Hyung Keun, 06004 Seoul (KR)
(74) Representative: Mewburn Ellis LLP

(57) **Abstract**

The present invention relates to a composition for diagnosis of pancreatic diseases, more specifically, to a composition which can diagnose pancreatic cancer, a kit for diagnosis comprising the same, and a method of providing information for diagnosis using the composition.

The present invention can accurately predict or identify the risk of pancreatic cancer, and moreover, can diagnose pancreatic cancer by effectively distinguishing pancreatic cancer from different types of cancer. In addition, in the present invention, it is possible to diagnose pancreatic cancer simply and rapidly through a non-invasive method by using monocytes obtained from blood, serum or plasma obtained from an individual.

<110>    Industry-Academic Cooperation Foundation, Yonsei University

<120>    Composition for diagnosing disease

<130>    DPB163663.k01

<150>    KR 10-2016-0157502
<151>    2016-11-24

<160>    5

<170>    KoPatentIn 3.0

<210>    1
<211>    325
<212>    PRT
<213>    Homo sapiens

<400>    1
Met Ala Trp Ser Leu Gly Ser Trp Leu Gly Gly Cys Leu Leu Val Ser
  1               5                  10                  15

Ala Leu Gly Met Val Pro Pro Pro Glu Asn Val Arg Met Asn Ser Val
             20                  25                  30

Asn Phe Lys Asn Ile Leu Gln Trp Glu Ser Pro Ala Phe Ala Lys Gly
         35                  40                  45

Asn Leu Thr Phe Thr Ala Gln Tyr Leu Ser Tyr Arg Ile Phe Gln Asp
     50                  55                  60

Lys Cys Met Asn Thr Thr Leu Thr Glu Cys Asp Phe Ser Ser Leu Ser
 65                  70                  75                  80

Lys Tyr Gly Asp His Thr Leu Arg Val Arg Ala Glu Phe Ala Asp Glu
                 85                  90                  95

His Ser Asp Trp Val Asn Ile Thr Phe Cys Pro Val Asp Asp Thr Ile
            100                 105                 110

Ile Gly Pro Pro Gly Met Gln Val Glu Val Leu Ala Asp Ser Leu His
        115                 120                 125

Met Arg Phe Leu Ala Pro Lys Ile Glu Asn Glu Tyr Glu Thr Trp Thr
    130                 135                 140

Met Lys Asn Val Tyr Asn Ser Trp Thr Tyr Asn Val Gln Tyr Trp Lys
145                 150                 155                 160

Asn Gly Thr Asp Glu Lys Phe Gln Ile Thr Pro Gln Tyr Asp Phe Glu
                165                 170                 175

Val Leu Arg Asn Leu Glu Pro Trp Thr Thr Tyr Cys Val Gln Val Arg
            180                 185                 190

Gly Phe Leu Pro Asp Arg Asn Lys Ala Gly Glu Trp Ser Glu Pro Val
        195                 200                 205

Cys Glu Gln Thr Thr His Asp Glu Thr Val Pro Ser Trp Met Val Ala
    210                 215                 220

Val Ile Leu Met Ala Ser Val Phe Met Val Cys Leu Ala Leu Leu Gly
225                 230                 235                 240

Cys Phe Ala Leu Leu Trp Cys Val Tyr Lys Lys Thr Lys Tyr Ala Phe
                245                 250                 255

Ser Pro Arg Asn Ser Leu Pro Gln His Leu Lys Glu Phe Leu Gly His
            260                 265                 270

Pro His His Asn Thr Leu Leu Phe Phe Ser Phe Pro Leu Ser Asp Glu
        275                 280                 285

Asn Asp Val Phe Asp Lys Leu Ser Val Ile Ala Glu Asp Ser Glu Ser
    290                 295                 300

Gly Lys Gln Asn Pro Gly Asp Ser Cys Ser Leu Gly Thr Pro Pro Gly
305                 310                 315                 320

Gln Gly Pro Gln Ser
                325


<210>    2
<211>    574
<212>    PRT
<213>    Homo sapiens

<400>    2
Met Arg Thr Leu Leu Thr Ile Leu Thr Val Gly Ser Leu Ala Ala His
  1               5                  10                  15

Ala Pro Glu Asp Pro Ser Asp Leu Leu Gln His Val Lys Phe Gln Ser
             20                  25                  30

Ser Asn Phe Glu Asn Ile Leu Thr Trp Asp Ser Gly Pro Glu Gly Thr
         35                  40                  45

Pro Asp Thr Val Tyr Ser Ile Glu Tyr Lys Thr Tyr Gly Glu Arg Asp
     50                  55                  60

Trp Val Ala Lys Lys Gly Cys Gln Arg Ile Thr Arg Lys Ser Cys Asn
 65                  70                  75                  80

Leu Thr Val Glu Thr Gly Asn Leu Thr Glu Leu Tyr Tyr Ala Arg Val
                 85                  90                  95

Thr Ala Val Ser Ala Gly Gly Arg Ser Ala Thr Lys Met Thr Asp Arg
            100                 105                 110

Phe Ser Ser Leu Gln His Thr Thr Leu Lys Pro Pro Asp Val Thr Cys
        115                 120                 125

Ile Ser Lys Val Arg Ser Ile Gln Met Ile Val His Pro Thr Pro Thr
    130                 135                 140

Pro Ile Arg Ala Gly Asp Gly His Arg Leu Thr Leu Glu Asp Ile Phe
145                 150                 155                 160

His Asp Leu Phe Tyr His Leu Glu Leu Gln Val Asn Arg Thr Tyr Gln
                165                 170                 175

Met His Leu Gly Gly Lys Gln Arg Glu Tyr Glu Phe Phe Gly Leu Thr
            180                 185                 190

Pro Asp Thr Glu Phe Leu Gly Thr Ile Met Ile Cys Val Pro Thr Trp

        195                 200                 205

Ala Lys Glu Ser Ala Pro Tyr Met Cys Arg Val Lys Thr Leu Pro Asp
    210                 215                 220

Arg Thr Trp Thr Tyr Ser Phe Ser Gly Ala Phe Leu Phe Ser Met Gly
225                 230                 235                 240

Phe Leu Val Ala Val Leu Cys Tyr Leu Ser Tyr Arg Tyr Val Thr Lys
                245                 250                 255

Pro Pro Ala Pro Pro Asn Ser Leu Asn Val Gln Arg Val Leu Thr Phe
            260                 265                 270

Gln Pro Leu Arg Phe Ile Gln Glu His Val Leu Ile Pro Val Phe Asp
        275                 280                 285

Leu Ser Gly Pro Ser Ser Leu Ala Gln Pro Val Gln Tyr Ser Gln Ile
    290                 295                 300

Arg Val Ser Gly Pro Arg Glu Pro Ala Gly Ala Pro Gln Arg His Ser
305                 310                 315                 320

Leu Ser Glu Ile Thr Tyr Leu Gly Gln Pro Asp Ile Ser Ile Leu Gln
                325                 330                 335

Pro Ser Asn Val Pro Pro Pro Gln Ile Leu Ser Pro Leu Ser Tyr Ala
            340                 345                 350

Pro Asn Ala Ala Pro Glu Val Gly Pro Pro Ser Tyr Ala Pro Gln Val
        355                 360                 365

Thr Pro Glu Ala Gln Phe Pro Phe Tyr Ala Pro Gln Ala Ile Ser Lys
    370                 375                 380

Val Gln Pro Ser Ser Tyr Ala Pro Gln Ala Thr Pro Asp Ser Trp Pro
385                 390                 395                 400

Pro Ser Tyr Gly Val Cys Met Glu Gly Ser Gly Lys Asp Ser Pro Thr
                405                 410                 415

Gly Thr Leu Ser Ser Pro Lys His Leu Arg Pro Lys Gly Gln Leu Gln
            420                 425                 430

Lys Glu Pro Pro Ala Gly Ser Cys Met Leu Gly Gly Leu Ser Leu Gln
        435                 440                 445

Glu Val Thr Ser Leu Ala Met Glu Glu Ser Gln Glu Ala Lys Ser Leu
    450                 455                 460

His Gln Pro Leu Gly Ile Cys Thr Asp Arg Thr Ser Asp Pro Asn Val
465                 470                 475                 480

Leu His Ser Gly Glu Glu Gly Thr Pro Gln Tyr Leu Lys Gly Gln Leu
                485                 490                 495

Pro Leu Leu Ser Ser Val Gln Ile Glu Gly His Pro Met Ser Leu Pro
            500                 505                 510

Leu Gln Pro Pro Ser Arg Pro Cys Ser Pro Ser Asp Gln Gly Pro Ser
        515                 520                 525

Pro Trp Gly Leu Leu Glu Ser Leu Val Cys Pro Lys Asp Glu Ala Lys

530                 535                 540

Ser Pro Ala Pro Glu Thr Ser Asp Leu Glu Gln Pro Thr Glu Leu Asp
545                 550                 555                 560

Ser Leu Phe Arg Gly Leu Ala Leu Thr Val Gln Trp Glu Ser
                565                 570

<210>    3
<211>    179
<212>    PRT
<213>    Homo sapiens

<400>    3
Met Ala Ala Leu Gln Lys Ser Val Ser Ser Phe Leu Met Gly Thr Leu
  1               5                  10                  15

Ala Thr Ser Cys Leu Leu Leu Leu Ala Leu Leu Val Gln Gly Gly Ala
             20                  25                  30

Ala Ala Pro Ile Ser Ser His Cys Arg Leu Asp Lys Ser Asn Phe Gln
         35                  40                  45

Gln Pro Tyr Ile Thr Asn Arg Thr Phe Met Leu Ala Lys Glu Ala Ser
     50                  55                  60

Leu Ala Asp Asn Asn Thr Asp Val Arg Leu Ile Gly Glu Lys Leu Phe
 65                  70                  75                  80

His Gly Val Ser Met Ser Glu Arg Cys Tyr Leu Met Lys Gln Val Leu
                 85                  90                  95

Asn Phe Thr Leu Glu Glu Val Leu Phe Pro Gln Ser Asp Arg Phe Gln
            100                 105                 110

Pro Tyr Met Gln Glu Val Val Pro Phe Leu Ala Arg Leu Ser Asn Arg
        115                 120                 125

Leu Ser Thr Cys His Ile Glu Gly Asp Asp Leu His Ile Gln Arg Asn
    130                 135                 140

Val Gln Lys Leu Lys Asp Thr Val Lys Lys Leu Gly Glu Ser Gly Glu
145                 150                 155                 160

Ile Lys Ala Ile Gly Glu Leu Asp Leu Leu Phe Met Ser Leu Arg Asn
                165                 170                 175

Ala Cys Ile

<210>    4
<211>    200
<212>    PRT
<213>    Homo sapiens

<400>    4
Met Ala Ala Ala Trp Thr Val Val Leu Val Thr Leu Val Leu Gly Leu
  1               5                  10                  15

Ala Val Ala Gly Pro Val Pro Thr Ser Lys Pro Thr Thr Thr Gly Lys
             20                  25                  30

Gly Cys His Ile Gly Arg Phe Lys Ser Leu Ser Pro Gln Glu Leu Ala
            35                  40                  45

Ser Phe Lys Lys Ala Arg Asp Ala Leu Glu Glu Ser Leu Lys Leu Lys
        50                  55                  60

Asn Trp Ser Cys Ser Ser Pro Val Phe Pro Gly Asn Trp Asp Leu Arg
65                  70                  75                  80

Leu Leu Gln Val Arg Glu Arg Pro Val Ala Leu Glu Ala Glu Leu Ala
                85                  90                  95

Leu Thr Leu Lys Val Leu Glu Ala Ala Ala Gly Pro Ala Leu Glu Asp
            100                 105                 110

Val Leu Asp Gln Pro Leu His Thr Leu His His Ile Leu Ser Gln Leu
        115                 120                 125

Gln Ala Cys Ile Gln Pro Gln Pro Thr Ala Gly Pro Arg Pro Arg Gly
    130                 135                 140

Arg Leu His His Trp Leu His Arg Leu Gln Glu Ala Pro Lys Lys Glu
145                 150                 155                 160

Ser Ala Gly Cys Leu Glu Ala Ser Val Thr Phe Asn Leu Phe Arg Leu
                165                 170                 175

Leu Thr Arg Asp Leu Lys Tyr Val Ala Asp Gly Asn Leu Cys Leu Arg
            180                 185                 190

Thr Ser Thr His Pro Glu Ser Thr
        195                 200


<210>    5
<211>    520
<212>    PRT
<213>    Homo sapiens

<400>    5
Met Ala Gly Pro Glu Arg Trp Gly Pro Leu Leu Leu Cys Leu Leu Gln
1               5                   10                  15

Ala Ala Pro Gly Arg Pro Arg Leu Ala Pro Pro Gln Asn Val Thr Leu
            20                  25                  30

Leu Ser Gln Asn Phe Ser Val Tyr Leu Thr Trp Leu Pro Gly Leu Gly
        35                  40                  45

Asn Pro Gln Asp Val Thr Tyr Phe Val Ala Tyr Gln Ser Ser Pro Thr
    50                  55                  60

Arg Arg Arg Trp Arg Glu Val Glu Glu Cys Ala Gly Thr Lys Glu Leu
65                  70                  75                  80

Leu Cys Ser Met Met Cys Leu Lys Lys Gln Asp Leu Tyr Asn Lys Phe
                85                  90                  95

Lys Gly Arg Val Arg Thr Val Ser Pro Ser Ser Lys Ser Pro Trp Val
            100                 105                 110

Glu Ser Glu Tyr Leu Asp Tyr Leu Phe Glu Val Glu Pro Ala Pro Pro

        115                 120                 125

Val Leu Val Leu Thr Gln Thr Glu Glu Ile Leu Ser Ala Asn Ala Thr
    130                 135                 140

Tyr Gln Leu Pro Pro Cys Met Pro Pro Leu Asp Leu Lys Tyr Glu Val
145                 150                 155                 160

Ala Phe Trp Lys Glu Gly Ala Gly Asn Lys Thr Leu Phe Pro Val Thr
                165                 170                 175

Pro His Gly Gln Pro Val Gln Ile Thr Leu Gln Pro Ala Ala Ser Glu
            180                 185                 190

His His Cys Leu Ser Ala Arg Thr Ile Tyr Thr Phe Ser Val Pro Lys
        195                 200                 205

Tyr Ser Lys Phe Ser Lys Pro Thr Cys Phe Leu Leu Glu Val Pro Glu
    210                 215                 220

Ala Asn Trp Ala Phe Leu Val Leu Pro Ser Leu Leu Ile Leu Leu Leu
225                 230                 235                 240

Val Ile Ala Ala Gly Gly Val Ile Trp Lys Thr Leu Met Gly Asn Pro
                245                 250                 255

Trp Phe Gln Arg Ala Lys Met Pro Arg Ala Leu Asp Phe Ser Gly His
            260                 265                 270

Thr His Pro Val Ala Thr Phe Gln Pro Ser Arg Pro Glu Ser Val Asn
        275                 280                 285

Asp Leu Phe Leu Cys Pro Gln Lys Glu Leu Thr Arg Gly Val Arg Pro
    290                 295                 300

Thr Pro Arg Val Arg Ala Pro Ala Thr Gln Gln Thr Arg Trp Lys Lys
305                 310                 315                 320

Asp Leu Ala Glu Asp Glu Glu Glu Glu Asp Glu Glu Asp Thr Glu Asp
                325                 330                 335

Gly Val Ser Phe Gln Pro Tyr Ile Glu Pro Pro Ser Phe Leu Gly Gln
            340                 345                 350

Glu His Gln Ala Pro Gly His Ser Glu Ala Gly Gly Val Asp Ser Gly
        355                 360                 365

Arg Pro Arg Ala Pro Leu Val Pro Ser Glu Gly Ser Ser Ala Trp Asp
    370                 375                 380

Ser Ser Asp Arg Ser Trp Ala Ser Thr Val Asp Ser Ser Trp Asp Arg
385                 390                 395                 400

Ala Gly Ser Ser Gly Tyr Leu Ala Glu Lys Gly Pro Gly Gln Gly Pro
                405                 410                 415

Gly Gly Asp Gly His Gln Glu Ser Leu Pro Pro Pro Glu Phe Ser Lys
            420                 425                 430

Asp Ser Gly Phe Leu Glu Glu Leu Pro Glu Asp Asn Leu Ser Ser Trp
        435                 440                 445

Ala Thr Trp Gly Thr Leu Pro Pro Glu Pro Asn Leu Val Pro Gly Gly

450                 455                 460

Pro Pro Val Ser Leu Gln Thr Leu Thr Phe Cys Trp Glu Ser Ser Pro
465                 470                 475                 480

Glu Glu Glu Glu Glu Ala Arg Glu Ser Glu Ile Glu Asp Ser Asp Ala
                485                 490                 495

Gly Ser Trp Gly Ala Glu Ser Thr Gln Arg Thr Glu Asp Arg Gly Arg
            500                 505                 510

Thr Leu Gly His Tyr Met Ala Arg
        515                 520

## Description

### [Technical Field]

The present invention relates to a composition for diagnosis of a variety of diseases such as pancreatic cancer, a kit for diagnosis containing the same, and a method of providing information for diagnosis using the composition.

### [Background Art]

Studies on methods for the treatment and diagnosis of cancer, one of the major diseases of modern people, are actively underway, and are focused on lung cancer, liver cancer and stomach cancer, which have high incidence. However, studies on esophageal cancer, colorectal cancer, pancreatic cancer and the like, which have lower incidence, are relatively insufficient. In particular, pancreatic cancer exhibits almost no symptoms in an initial stage, but generally exhibits symptoms such as pain and weight loss after systemic metastasis. Therefore, pancreatic cancer has a lower recovery rate, so regular diagnosis therefor is very important. Most clinical symptoms develop slowly, and appetite decline, fatigue and weight loss are the most common symptoms. Pancreatic cancer is a fatal human cancer that has the worst prognosis, with a 5-year survival rate of 1 to 4% and a median survival rate of 5 months. In addition, 80% to 90% of pancreatic cancer patients are found, upon diagnosis, in a condition in which radical resection, capable of enabling complete recovery from pancreatic cancer, is not possible. For this reason, the prognosis of pancreatic cancer patients is poor, and the treatment thereof depends mainly on chemotherapy. Thus, first of all, there is an increasingly urgent need for the development of methods for early diagnosis of pancreatic cancer, more than for any other kind of human cancer.

The therapeutic efficacy of several anticancer drugs, including 5-fluorouracil, gemcitabine and Tarceva, which are currently known to be effective against pancreatic cancer, is extremely poor, and the rate of response to chemotherapy is low, *i.e.*, about 15%. This suggests that more effective early diagnosis and treatment methods are urgently needed in order to improve the prognosis of patients with pancreatic cancer.

Diagnosis of pancreatic cancer has to date been performed by x-ray radiography of the stomach and duodenum, cholangiography via the skin and liver, endoscopic retrograde cholangiography or the like, but recently, ultrasonography and computed tomography have come to be most commonly used. When a lesion of a disease is detected using any of these methods, more accurate examination results can be obtained by performing more precise biopsy. However, patients are reluctant to participate in such diagnostic methods due to the low accuracy thereof or considerable discomfort associated therewith, such as pain of patients. Therefore, there has been a need for the development of examination methods capable of simply and rapidly diagnosing pancreatic cancer.

In this regard, Korean Patent No. 10-0819122 discloses a technique using matrilin, transthyretin and stratifin as pancreatic cancer markers, and Korean Patent Publication No. 2012-0082372 discloses a technique using various pancreatic cancer markers. In addition, Korean Patent Publication No. 2009-0003308 discloses a method for diagnosing pancreatic cancer by detecting the amount of REG4 protein expressed in a blood sample from a subject, Korean Patent Publication No. 2012-0009781 discloses an assay method including measuring the amount of XIST RNA expressed in cancer tissue isolated from a subject in order to provide information required for the diagnosis of pancreatic cancer, Korean Patent Publication No. 2007-0119250 discloses a novel gene LBFL313 family differentially expressed in human pancreatic cancer tissue compared to pancreatic tissue of normal human, and U.S. Patent Publication No. 2011/0294136 discloses a method for diagnosing pancreatic cancer using biomarkers such as keratin 8 protein. However, the identification of markers with better effects is needed, since there is a great difference in diagnostic efficiency and accuracy between the markers.

### [Disclosure]

### [Technical Problem]

It is one object of the present invention to provide a composition capable of simply and accurately diagnosing diseases such as pancreatic diseases, autoimmune diseases, allergies, Grave's disease, Hashimoto's thyroiditis, autoimmune lymphoproliferative syndrome (ALPS), myasthenia gravis, Kawasaki disease or psoriasis, preferably pancreatic cancer, by measuring the expression levels of proteins of interleukin 10 receptor (IL-10R), interleukin 22 receptor (IL-22R), interleukin 22 (IL-22), interleukin 29 (IL-29), or interferon lambda receptor 1 isoform 1 (IFNLR1 isoform 1), or the expression levels of the mRNA of genes encoding the proteins.

It is another object of the present invention to provide a kit capable of simply and accurately diagnosing diseases such as pancreatic diseases, autoimmune diseases, allergies, Grave's disease, Hashimoto's thyroiditis, autoimmune lymphoproliferative syndrome (ALPS), myasthenia gravis, Kawasaki disease or psoriasis, preferably pancreatic cancer, by measuring the expression levels of proteins of an interleukin 10 receptor (IL-10R), an interleukin 22 receptor (IL-22R), interleukin 22 (IL-22), interleukin 29 (IL-29), or interferon lambda receptor 1 isoform 1 (IFNLR1 isoform 1), or the mRNA of genes encoding the proteins.

It is another object of the present invention to provide a method of providing information for diagnosing of a diseases such as pancreatic diseases, autoimmune diseases, allergies, Grave's disease, Hashimoto's thyroiditis, autoimmune lymphoproliferative syndrome (ALPS), myasthenia gravis, Kawasaki disease or psoriasis, preferably pancreatic cancer, by measuring the expression levels of proteins of an interleukin 10 receptor (IL-10R), an interleukin 22 receptor (IL-22R), interleukin 22 (IL-22), interleukin 29 (IL-29), or interferon lambda receptor 1 isoform 1 (IFNLR1 isoform 1), or the mRNA of genes encoding the proteins.

### [Technical Solution]

Pancreatic cancer exhibits almost no symptoms in an initial stage, but generally exhibits symptoms such as pain and weight loss after systemic metastasis. Therefore, pancreatic cancer has a lower recovery rate, so regular diagnosis therefor is very important. Most clinical symptoms develop slowly, and appetite decline, fatigue and weight loss are the most common symptoms. Pancreatic cancer is a fatal human cancer that has the worst prognosis, with a 5-year survival rate of 1 to 4% and a median survival rate of 5 months. In addition, 80% to 90% of pancreatic cancer patients are found, upon diagnosis, in a condition in which radical resection, capable of enabling complete recovery from pancreatic cancer, is not possible. For this reason, the prognosis of pancreatic cancer patients is poor, and the treatment thereof depends mainly on chemotherapy. Thus, first of all, there is an increasingly urgent need for the development of methods for early diagnosis of pancreatic cancer, more than for any other kind of human cancer.

Accordingly, as the result of thorough and extensive efforts to develop markers useful for the early diagnosis of pancreatic cancer, the present inventors have found that the expression of an interleukin 10 receptor (IL-10R), an interleukin 22 receptor (IL-22R), interleukin 22 (IL-22), interleukin 29 (IL-29), or interferon lambda receptor 1 isoform 1 (IFNLR1 isoform 1) is specifically increased in blood-derived mononuclear cells isolated from pancreatic cancer patients. Based on this finding, the present invention has been completed.

In accordance with one aspect of the present invention, there is provided a composition for diagnosing a disease comprising an agent for measuring the expression level of at least one protein selected from the group consisting of an interleukin 10 receptor (IL-10R), an interleukin 22 receptor (IL-22R), interleukin 22 (IL-22), interleukin 29 (IL-29), and interferon lambda receptor 1 isoform 1 (IFNLR1 isoform 1), or the expression level of the mRNA of a gene encoding the protein.

Examples of the disease that can be applied to the prediction of the onset or onset likelihood thereof using the composition for diagnosis of the present invention include, but are not limited to, pancreatic diseases, autoimmune diseases, allergies, Grave's disease, Hashimoto's thyroiditis, autoimmune lymphoproliferative syndrome (ALPS), myasthenia gravis, Kawasaki disease, psoriasis, and the like.

In particular, the present invention makes it possible to diagnose pancreatic cancer or pancreatitis, as pancreatic diseases, preferably pancreatic cancer, using the composition for diagnosis. Specifically, the composition for diagnosis of the present invention is capable of detecting or diagnosing a pancreatic cancer patient group, distinct from a normal group. In addition, the composition for diagnosis of the present invention is capable of selectively detecting or diagnosing pancreatic cancer, distinct from other types of cancer such as lung cancer or colorectal cancer.

As used herein, the term "pancreatic cancer" refers to cancer originating from pancreatic cells. There are a variety of kinds of pancreatic cancer, including pancreatic ductal adenocarcinoma of pancreatic duct, which accounts for about 90% of pancreatic cancer cases. Thus, "pancreatic cancer" typically means pancreatic ductal adenocarcinoma. In addition, there are cystadenocarcinoma, endocrine tumor and the like. About 5% to 10% of pancreatic cancer patients have a genetic predisposition thereto. The proportion of pancreatic cancer patients that have family history of pancreatic cancer is about 7.8%, which is higher than 0.6%, the proportion of pancreatic cancer onset in normal people. Pancreatic cancer has a very poor prognosis, with a 5-year survival rate of less than 5%. The reason for this is that most cases are detected after the cancer has progressed, so surgical resection is possible in less than 20% of cases at the time of detection, and even if the cancer is determined to be completely resected by visual examination, the increase in survival rate is low due to micro metastasis, and responsiveness to chemotherapy and radiotherapy is low. Therefore, the most important method for improving the survival rate is early detection and operation when symptoms are absent or nonspecific.

As used herein, the term "pancreatitis" refers to a disease caused by inflammation of the pancreas, and includes acute pancreatitis and chronic pancreatitis. The pancreatic juice contains digestive enzymes such as amylase (acting on carbohydrate hydrolysis), trypsin (acting on protein hydrolysis) and lipase (acting on lipid hydrolysis). Pancreatitis results from various causes such as metabolic disturbances, drugs, and abdominal injury, as well as self-degradation of the pancreas induced by the enzymes due to defective flow of pancreatic juice attributable to alcohol abuse, gallstones and the like. Pancreatitis is an inflammatory disease of the pancreas that induces damage to pancreatic duct cells, extensive interstitial edema, and migration of neutrophil granulocytes to haemorrhage and injury sites. Pancreatitis can be broadly divided into two types: a mild type of pancreatitis wherein interstitial edema and peripancreatic fat necrosis are detected, and a severe type of pancreatitis wherein extensive peripancreatic and intrapancreatic fat necrosis and pancreatic parenchymal necrosis are detected, accompanied by hemorrhaging (Bank PA., Am. J. gastroenterol., 89, pp 151-152, 1994.; Bradley EL., Arch. Surg., 128, pp 586-590, 1993.; Kim Chang Duk, Korean Journal of Gastroenterology, 46, pp 321-332, 2005).

Further, as used herein, the term "autoimmune disease" refers to a non-malignant disease or disorder that is generated and specified for subject's own tissues. One of the most important properties in all normal subjects is that they do not respond negatively to an antigenic substance that constitutes the self, but they can recognize non-self antigens, respond thereto and remove the same. The unresponsiveness of the organism to a self-antigen is called "immunologic unresponsiveness" or "tolerance". However, when an abnormality occurs in induction or maintenance of self-tolerance, an immune response to the self-antigen occurs, and as a result, a phenomenon of attack to self-tissue occurs. The disease caused by the aforementioned process is called "autoimmune disease". The autoimmune disease is an inflammatory disease in which an antibody is produced against a patient's own organ tissue or ingredient, and can be generally referred to as a disease that causes chronic systemic inflammation in many tissues and organs.

Specifically, the autoimmune diseases according to the present invention include, but are not limited to, rheumatoid arthritis (SLE), systemic lupus erythematosus, Crohn's disease, ulcerative colitis, multiple sclerosis, uveitis, autoimmune meningitis, Sjogren's syndrome, scleroderma, Wegener's granulomatosis, sarcoidosis, septic shock, dacryoadenitis, stroke, arteriosclerosis, vascular restenosis, type I diabetes, type II diabetes, urticaria, conjunctivitis, psoriasis, systemic inflammatory syndrome, multiple myositis, dermatomyositis, nodular polyarticular arthritis, mixed connective tissue disease, gout, Parkinson's disease, amyotrophic lateral sclerosis, diabetic retinopathy, chronic thyroiditis, celiac disease, myasthenia gravis, vesical pemphigus, viral diseases, bacterial diseases, arteriosclerosis, angioma, angiofibroma, reperfusion injury, cardiac hypertrophy and the like.

As used herein, the term "allergy" may be used interchangeably with "atopic disorder" and may refer to a disease caused by a biochemical phenomenon that shows a specific and modified response to foreign substance, that is, an allergen.

In the present invention, the allergy may include atopic dermatitis, contact dermatitis, eczema, asthma, hypersensitivity, allergic rhinitis, allergic conjunctivitis, allergic dermatitis, urticaria, insect allergies, food allergies, drug allergies and the like, but is not limited thereto.

In the present invention, the term "diagnosis" is intended to include determining the susceptibility of a subject to a particular disease or disorder, determining whether the subject has a particular disease or disorder at present, determining the prognosis of the subject having a particular disease or disorder (e.g., identifying a pre-metastatic or metastatic cancerous condition, determining the stage of the cancer or determining the response of the cancer to treatment), or therametrics (e.g., monitoring the status of the subject in order to provide information for therapeutic effects). Regarding the objects of the present invention, the diagnosis is to determine the onset or likelihood (risk) of onset of the disease.

The composition for diagnosis of the present invention may preferably contain an agent for measuring the expression level of an interleukin 10 receptor (IL-10R) or mRNA of a gene encoding the interleukin 10 receptor.

As used herein, the term "interleukin 10 receptor (IL-10R)" refers to a type II cytokine receptor which corresponds to a tetramer consisting of two α (alpha) subunits and two β (beta) subunits. The α subunit is expressed in hematopoietic cells such as T cells, B cells, NK cells, mast cells and dendritic cells, and the β subunit is expressed in various ways. In the present invention, the interleukin 10 receptor is preferably a β subunit of the interleukin 10 receptor (IL-10RB) represented by SEQ ID NO: 1.

In addition, the composition for diagnosis of the present invention further contains an agent for measuring the expression level of an interleukin 22 receptor (IL-22R) or mRNA of a gene encoding the interleukin 22 receptor, thereby improving the accuracy of diagnosis of the disease.

As used herein, the term "interleukin 22 receptor (IL-22R)" refers to a type II cytokine receptor which corresponds to a heterodimer of an α1 subunit and a β2 subunit and binds to interleukin 22. In the present invention, the interleukin 22 receptor may be an interleukin 22 receptor α1 subunit (IL-22RA1) represented by SEQ ID NO: 2.

In addition, the composition for diagnosis of the present invention further contains an agent for measuring the expression level of at least one protein selected from the group consisting of interleukin 22 (IL-22), interleukin 29 (IL-29) and interferon lambda receptor 1 isoform 1 (IFNLR1 isoform 1) or mRNA of a gene encoding the protein, thereby improving the accuracy of diagnosis of the disease.

As used herein, the term "interleukin" 22 (IL-22)" refers to cytokine belonging to a cytokine group called "IL-10 family" or "IL-10 superfamily" (IL-19, IL-20, IL-24 and IL-26), which mediates a cellular immune response. The interleukin 22 binds to heterodimeric cell surface receptors consisting of subunits of IL-10R2 and IL-22R1. In the present invention, the interleukin 22 may be represented by the amino acid sequence of SEQ ID NO: 3.

As used herein, the term "interleukin 29 (IL-29)", which is also called "interferon lambda-1", refers to a protein encoded by interleukin 29 gene located on chromosome 19, belongs to the helical cytokine family, and corresponds to type III interferon. The interleukin 29 plays a key role in host defenses against microorganisms, and its expression level is greatly increased in virus-infected cells. In the present invention, the interleukin 29 may be represented by the amino acid sequence of SEQ ID NO: 4.

As used herein, the term "interferon lambda receptor 1 (IFNLR1)" refers to a protein encoded by a gene belonging to the type II cytokine receptor family, and binds to an interleukin 10 receptor beta subunit to form a receptor complex. The receptor complex can interact with interleukin 28A, interleukin 28B, and interleukin 29. In the present invention, the interferon lambda receptor 1 may be interferon lambda receptor 1 isoform 1 (IFNLR1 isoform 1), represented by the amino acid sequence of SEQ ID NO: 5.

In the present invention, the protein of the interleukin 10 receptor, interleukin 22 receptor, interleukin 22, interleukin 29, or interferon lambda receptor 1 isoform 1, or the mRNA of the gene encoding the protein may be present in a biopsy specimen, preferably blood, serum or plasma isolated from a target subject, and more preferably a mononuclear cell or exosome isolated from the blood, serum or plasma.

In conventional methods for measuring the expression level of a biomarker for diagnosis of various diseases, particularly pancreatic diseases, cells are isolated from the pancreatic tissue and the expression level of the biomarker in the cells is measured. Such a method has disadvantages of inconvenience associated with separation of cells from the pancreatic tissue for the diagnosis of pancreatic disease, preferably pancreatic cancer, and near impossibility of early diagnosis of pancreatic cancer due to the absence of observable symptoms in the initial stage of pancreatic cancer.

However, the present invention makes it possible to rapidly and simply, yet very accurately diagnose the onset of various diseases including pancreatic cancer and the likelihood of onset thereof by measuring the expression level of the interleukin 10 receptor, interleukin 22 receptor, interleukin 22, interleukin 29, or interferon lambda receptor 1 isoform 1, or the mRNA of the gene thereof in the mononuclear cells or exosomes contained in the blood, serum or plasma isolated from the target subject.

Specifically, when the expression level of the marker according to the present invention is measured in mononuclear cells or exosomes isolated from the target subject as described above, the onset of disease or the likelihood of the onset thereof can be rapidly and simply diagnosed, since there is no need for invasive procedures, for example, including conducing laparotomy on a patient and separating tissue cells from tissue (for example, pancreatic tissue), and it takes about 5 minutes or less to obtain a biopsy specimen including the mononuclear cell or exosome and about 2 hours or less to measure the expression levels of various disease biomarkers according to the present invention from the mononuclear cell or exosome.

In the present invention, the agent for measuring the expression level of the interleukin 10 receptor, interleukin 22 receptor, interleukin 22, interleukin 29, or interferon lambda receptor 1 isoform 1 is not particularly limited, but preferably includes at least one selected from the group consisting of antibodies, oligopeptides, ligands, PNAs (peptide nucleic acids) and aptamers that specifically bind to respective proteins of the interleukin 10 receptor, interleukin 22 receptor, interleukin 22, interleukin 29, or interferon lambda receptor 1 isoform 1.

As used herein, the term "measurement of expression levels of proteins" refers to a process of detecting the presence of proteins of interleukin 10 receptor, interleukin 22 receptor, interleukin 22, interleukin 29, or interferon lambda receptor 1 isoform 1, which is a marker for the diagnosis of pancreatic diseases, and the expression level thereof in biological samples in order to diagnose diseases including pancreatic cancer of the present invention. The methods for measurement of the expression levels of receptors or comparative analysis thereof include, but are not limited to, protein chip analysis, immunoassay, ligand-binding assay, MALDI-TOF (matrix assisted laser desorption/ionization time of flight mass spectrometry) analysis, SELDI-TOF (surface enhanced laser desorption/ionization time of flight mass spectrometry) analysis, radiation immunoassay, radiation immunodiffusion, ouchterlony immunodiffusion, rocket immunoelectrophoresis, tissue immunostaining, complement fixation analysis, 2D electrophoresis analysis, liquid chromatography-mass spectrometry (LC-MS), liquid chromatography-mass spectrometry/mass spectrometry (LC-MS/MS), western blotting, enzyme-linked immunosorbent assay (ELISA) and the like.

As used herein, the term "antibody" refers to a substance that specifically binds to an antigen to induce an antigen-antibody reaction. Regarding the objects of the present invention, an antibody means an antibody that specifically binds to an interleukin 10 receptor, an interleukin 22 receptor, interleukin 22, interleukin 29, or interferon lambda receptor 1 isoform 1. The antibody of the present invention includes all of a polyclonal antibody, a monoclonal antibody and a recombinant antibody. The antibody can be easily produced using techniques that are well-known in the art. For example, a polyclonal antibody may be prepared by a method well-known in the art, including injecting an antigen of the interleukin 10 receptor, interleukin 22 receptor, interleukin 22, interleukin 29 or interferon lambda receptor 1 isoform 1 into an animal and collecting the blood from the animal to obtain a serum containing an antibody. The polyclonal antibody can be produced from any animals such as goats, rabbits, sheep, monkeys, horses, pigs, cows and dogs. In addition, the monoclonal antibody can be produced using hybridoma methods well-known in the art (see Kohler and Milstein (1976) European Journal of Immunology 6: 511-519) or phage antibody library techniques (see Clackson et al, Nature, 352: 624-628, 1991; Marks et al., J. Mol. Biol., 222: 58, 1-597, 1991). The antibody prepared by the method can be separated and purified by a method such as gel electrophoresis, dialysis, salt precipitation, ion exchange chromatography, or affinity chromatography. In addition, the antibody of the present invention includes not only a complete antibody having two full-length light chains and two full-length heavy chains, but also a functional fragment of the antibody molecule. The functional fragment of the antibody molecule means a fragment having at least an antigen-binding function, and includes Fab, F(ab'), F(ab')2, Fv and the like.

As used herein, the term "PNA (peptide nucleic acid)" refers to an artificially synthesized DNA- or RNA-like polymer, which was first introduced by the professors, Nielsen, Egholm, Berg and Buchardt, in University of Copenhagen, Denmark in 1991. DNA has a phosphate-ribose sugar backbone, but PNA has repeated N-(2-aminoethyl)-glycine backbone linked via peptide bonds, and thus provides greatly increased binding capacity to DNA or RNA and stability and thus is used for molecular biology, diagnostic assays and antisense therapies. PNA is disclosed in detail in the literature [Nielsen PE, Egholm M, Berg RH, Buchardt O (December 1991). "Sequence-selective recognition of DNA by strand displacement with a thymine-substituted polyamide". Science 254 (5037): 1497-1500].

As used herein, the term "aptamer" refers to an oligonucleotide or a peptide molecule, and the general contents of the aptamer are disclosed in detail in the literature [Bock LC et al., Nature 355(6360):5646(1992); Hoppe-Seyler F, Butz K "Peptide aptamers: powerful new tools for molecular medicine". J Mol Med. 78(8):42630(2000); Cohen BA, Colas P, Brent R. "An artificial cell-cycle inhibitor isolated from a combinatorial library". Proc. Natl. Acad. Sci. USA. 95(24): 14272-7(1998)] .

In the composition for diagnosis according to the present invention, the agent for measuring the expression level of the mRNA of the gene encoding each of the interleukin 10 receptor, interleukin 22 receptor, interleukin 22, interleukin 29 or interferon lambda receptor 1 isoform 1 may include at least one selected from the group consisting of primers, probes and antisense nucleotides that specifically bind to the mRNA of the gene encoding each of the interleukin 10 receptor, interleukin 22 receptor, interleukin 22, interleukin 29 or interferon lambda receptor 1 isoform 1. Since the information of the interleukin 10 receptor, the interleukin 22 receptor, the interleukin 22, the interleukin 29 and the interferon lambda receptor 1 isoform 1 according to the present invention is known, those skilled in the art can easily design primers, probes, or antisense nucleotides specifically binding to the mRNA of the gene encoding the protein based on the information.

As used herein, the term "measurement of expression levels of mRNA" refers to a process of detecting the presence of mRNA of genes encoding the interleukin 10 receptor, interleukin 22 receptor, interleukin 22, interleukin 29, or interferon lambda receptor 1 isoform 1, and the expression level thereof in biological samples in order to diagnose diseases including pancreatic cancer of the present invention, and means measurement of the amount of mRNA. The analysis methods for measurement of expression levels of mRNA include, but are not limited to, reverse transcription polymerase chain reaction (RT-PCR), competitive reverse transcription polymerase chain reaction (competitive RT-PCR), real-time RT-PCR, RNase protection assay (RPA), northern blotting, DNA chips and the like.

As used herein, the term "primer" refers to a fragment recognizing a target gene sequence, and includes a pair of primers in both forward and reverse, but is preferably a pair of primers providing specific and sensitive analysis results. High specificity can be imparted to a primer in which the nucleic acid thereof is a sequence inconsistent with the non-target sequence present in the sample, so that only the target gene sequence containing a complementary primer-binding site is amplified, and nonspecific amplification is not induced.

As used herein, the term "probe" refers to a substance capable of specifically binding to a target substance to be detected in the sample, and refers to a substance capable of specifically detecting the presence of the target substance in the sample through the binding. The type of probe is not particularly limited so long as it is commonly used in the art and is preferably PNA (peptide nucleic acid), LNA (locked nucleic acid), a peptide, a polypeptide, a protein, an RNA or a DNA, and is most preferably PNA. More specifically, the probe is a biomolecule derived from an organism or an analogue thereof, or is produced *in vitro* and includes, for example, an enzyme, a protein, an antibody, a microorganism, an animal and/or plant cell and organ, DNA and RNA, the DNA may include cDNA, genomic DNA, and oligonucleotides, the RNA may include genomic RNA, mRNA and oligonucleotides, and examples of the protein may include antibodies, antigens, enzymes, peptides, and the like.

As used herein, the term "LNA (locked nucleic acid)" means a nucleic acid analogue including 2'-O, 4'-C methylene bridges [J Weiler, J Hunziker and J Hall Gene Therapy (2006) 13, 496.502]. LNA nucleosides include the common nucleotide bases of DNA and RNA, and can form base pairs according to the Watson-Crick base-pair rule. However, LNA fails to form an ideal shape in the Watson-Crick bond due to "locking" of the molecule attributable to the methylene bridge. When LNAs are incorporated in DNA or RNA oligonucleotides, they can more rapidly pair with complementary nucleotide chains to enhance the stability of the double strand. As used herein, the term "antisense" means an oligomer that has a nucleotide base sequence and a backbone between subunits, wherein an antisense oligomer is hybridized with the target sequence in the RNA by Watson-Crick base pairing to allow the formation of the mRNA and RNA:oligomer heterodimers in the target sequence. The oligomer may have an accurate or approximate sequence complementarity to the target sequence.

In accordance with another aspect of the present invention, there is provided a kit for diagnosing diseases containing the composition for diagnosis according to the present invention.

Examples of the disease that can be applied to the prediction of the onset or likelihood of onset thereof using the kit for diagnosis of the present invention include, but are not limited to, pancreatic diseases, autoimmune diseases, allergies, Grave's disease, Hashimoto's thyroiditis, autoimmune lymphoproliferative syndrome (ALPS), myasthenia gravis, Kawasaki disease, psoriasis, and the like.

In particular, the present invention makes it possible to diagnose pancreatic cancer or pancreatitis, as pancreatic diseases, preferably pancreatic cancer, using the kit for diagnosis. Specifically, the kit of the present invention is capable of detecting or diagnosing a pancreatic cancer patient group, distinct from a normal group. In addition, the kit of the present invention is capable of selectively detecting or diagnosing pancreatic cancer, distinct from other types of cancer such as lung cancer or colorectal cancer.

In the present invention, the kit may be an RT-PCR kit, a DNA chip kit, an ELISA kit, a protein chip kit, a rapid kit, or a multiple reaction-monitoring (MRM) kit, but is not limited thereto.

The kit for diagnosing diseases of the present invention may further include one or more types of other component compositions, solutions or devices suitable for the analysis method.

For example, the kit for diagnosis may further include essential elements required for performing a reverse transcription polymerase chain reaction. The RT-PCR kit includes a primer pair specific for the gene encoding the marker protein. The primer is a nucleotide having a sequence specific for the nucleic acid sequence of the gene, and may have a length of about 7 bp to 50 bp, more preferably about 10 bp to 30 bp. The primer may also include a primer specific for the nucleic acid sequence of the control gene. In addition, the RT-PCR kit may include test tubes or other appropriate containers, reaction buffers (at various pHs and magnesium concentrations), deoxynucleotides (dNTPs), enzymes such as Taq-polymerase and reverse transcriptase, DNase and/or RNase inhibitors, DEPC water, sterile water, and the like.

In addition, the kit for diagnosis of the present invention may include essential elements required for operating DNA chips. The DNA chip kit may include a substrate to which a cDNA or oligonucleotide corresponding to a gene or a fragment thereof is attached, and a reagent, an agent, an enzyme, and the like for producing a fluorescent-labeled probe. The substrate may also include a cDNA or oligonucleotide corresponding to a control gene or fragment thereof.

In addition, the kit for diagnosis of the present invention can include essential elements required for performing ELISA. The ELISA kit includes an antibody specific for the protein. The antibody has high specificity and affinity for the marker protein and little cross-reactivity with other proteins, and is a monoclonal antibody, a polyclonal antibody or a recombinant antibody. The ELISA kit may also include antibodies specific for the control protein. Furthermore, the ELISA kit may include a reagent capable of detecting the bound antibody, such as a labeled secondary antibody, a chromophore, an enzyme (e.g., conjugated to an antibody) and substrate thereof, another substance that can bind to the antibody or the like.

In accordance with another aspect of the present invention, there is provided a method of providing information for diagnosis of a disease including measuring the expression level of at least one protein selected from the group consisting of an interleukin 10 receptor (IL-10R), an interleukin 22 receptor (IL-22R), interleukin 22 (IL-22), interleukin 29 (IL-29), or interferon lambda receptor 1 isoform 1 (IFNLR1 isoform 1), or the mRNA of a gene encoding the protein in a biopsy specimen isolated from a target subject.

Examples of the disease that can be applied to the prediction of the onset or likelihood of onset thereof in the method of providing information according to the present invention include, but are not limited to, pancreatic diseases, autoimmune diseases, allergies, Grave's disease, Hashimoto's thyroiditis, autoimmune lymphoproliferative syndrome (ALPS), myasthenia gravis, Kawasaki disease, psoriasis, and the like.

In particular, the present invention makes it possible to more accurately diagnose and predict the onset or likelihood of onset of the aforementioned diseases, particularly, pancreatic diseases, specifically, pancreatic cancer or pancreatitis, more preferably, pancreatic cancer.

Specifically, the method of providing information according to the present invention is capable of detecting or diagnosing a pancreatic cancer patient group, distinct from a normal group, and is capable of selectively detecting or diagnosing pancreatic cancer, distinct from other types of cancer such as lung cancer or colorectal cancer.

In the present invention, the specific types of the autoimmune diseases and allergies overlap with those described in the composition for diagnosis of the present invention, and a description thereof will be omitted below.

As used herein, the term "target subject" refers to a subject for which it is not certain whether the disease set forth above is developed, and which is highly likely to develop the disease.

As used herein, the term "biopsy specimen" refers to tissue for histopathological examination application, which is collected by inserting a hollow needle or the like into an organ of the organism, rather than cutting the skin of a patient with a high possibility of developing a disease and the biopsy specimen may include a patient's tissue, cells, blood, serum, plasma, saliva or sputum, preferably a patient's blood, serum or plasma, and more preferably a mononuclear cell or exosome isolated from the blood, serum or plasma.

In conventional methods for measuring the expression level of a biomarker for diagnosis of pancreatic diseases, cells are isolated from the tissue (for example, pancreatic tissue) in which it is predicted that a disease will develop, and the expression level of the biomarker in the cells is measured. However, the present invention makes it possible to rapidly and simply, yet very accurately diagnose the onset of various diseases including pancreatic cancer and the likelihood of the onset thereof by measuring the expression level of the disease biomarker according to the present invention in the mononuclear cell or exosome contained in the blood, serum or plasma isolated from the target subject.

Specifically, when the expression level of the marker according to the present invention is measured in the mononuclear cell or exosome isolated from the target subject as described above, the onset of a disease or the likelihood of the onset thereof can be very rapidly and simply diagnosed, since there is no need for invasive procedures, for example, including conducing laparotomy on a patient and separating tissue cells from tissue (for example, pancreatic tissue), and it takes about 5 minutes or less to obtain a biopsy specimen including the mononuclear cell, and about 2 hours or less to measure the expression level of the biomarker according to the present invention from the mononuclear cell.

The method of providing information according to the present invention is preferably capable of measuring, from the isolated biopsy specimen, the expression level of, as a biomarker, an interleukin 10 receptor or mRNA of the gene encoding the interleukin 10 receptor.

In addition, the method of providing information according to the present invention further includes measuring, from the biopsy specimen, the expression level of, as another biomarker for diagnosis of diseases including pancreatic cancer according to the present invention, an interleukin 22 receptor, or mRNA of a gene encoding the interleukin 22 receptor, thereby improving the accuracy of diagnosis of the disease.

In addition, the method of providing information according to the present invention further includes measuring, from the biopsy specimen, the expression level of, as yet another biomarker for diagnosis of diseases including pancreatic cancer according to the present invention, at least one protein selected from the group consisting of interleukin 22, interleukin 29 and interferon lambda receptor 1 isoform 1, or mRNA of a gene encoding the protein, thereby improving the accuracy of diagnosis of the disease.

The agent for measuring the expression level of the interleukin 10 receptor, the interleukin 22 receptor, interleukin 22, the interleukin 29, or the interferon lambda receptor 1 isoform 1 is not particularly limited, but preferably includes at least one selected from the group consisting of antibodies, oligopeptides, ligands, PNAs (peptide nucleic acids) and aptamers that specifically bind to each of the interleukin 10 receptor, the interleukin 22 receptor, the interleukin 22, the interleukin 29 or the interferon lambda receptor 1 isoform 1.

Preferably, in the present invention, the expression level of the interleukin 10 receptor, interleukin 22 receptor, interleukin 22, interleukin 29, or interferon lambda receptor 1 isoform 1 can be measured and compared using antibodies specifically binding to the interleukin 10 receptor, the interleukin 22 receptor, the interleukin 22, interleukin 29, or the interferon lambda receptor 1 isoform 1. This can be carried out using a method of detecting an antigen-antibody complex which is formed by the antibody with the corresponding protein in the biological sample.

As used herein, the term "antigen-antibody complex" refers to a combination of a protein antigen for detecting the presence or absence of the corresponding gene in the biological sample with an antibody recognizing the protein antigen. The detection of the antigen-antibody complex can be carried out using any of methods well-known in the art, for example, spectroscopic, photochemical, biochemical, immunochemical, electrical, absorption spectrometric, chemical and other methods.

Meanwhile, in the present invention, the methods for measurement of expression levels of proteins or comparative analysis thereof include, but are not limited to, protein chip analysis, immunoassay, ligand-binding assay, MALDI-TOF (matrix assisted laser desorption/ionization time of flight mass spectrometry) analysis, radiation immunoassay, radiation immunodiffusion, SELDI-TOF (surface enhanced laser desorption/ionization time of flight mass spectrometry) analysis, radiation immunoassay, radiation immunodiffusion, ouchterlony immunodiffusion, rocket immunoelectrophoresis, tissue immunostaining, complement fixation analysis, 2D electrophoresis analysis, liquid chromatography-mass spectrometry (LC-MS), liquid chromatography-mass spectrometry/mass spectrometry (LC-MS/MS), western blotting, enzyme-linked immunosorbent assay (ELISA) and the like.

In addition, in the present invention, the agent for measuring the expression level of the mRNA of the gene encoding each of the interleukin 10 receptor, the interleukin 22 receptor, the interleukin 22, the interleukin 29 or the interferon lambda receptor 1 isoform 1 may include at least one selected from the group consisting of primers, probes and antisense nucleotides that specifically bind to the mRNA of the gene encoding the protein of each of the interleukin 10 receptor, the interleukin 22 receptor, the interleukin 22, the interleukin 29 or the interferon lambda receptor 1 isoform 1. Since the information of the interleukin 10 receptor, the interleukin 22 receptor, the interleukin 22, the interleukin 29 and the interferon lambda receptor 1 isoform 1 according to the present invention is known, those skilled in the art can easily design primers, probes, or antisense nucleotides specifically binding to the mRNA of the gene encoding the protein based on the information.

In the present invention, the measurement and comparison of expression levels of the mRNA of the gene encoding the interleukin 10 receptor, the interleukin 22 receptor, the interleukin 22, the interleukin 29 or the interferon lambda receptor 1 isoform 1 may be carried out using a method including, but not limited to, reverse transcription polymerase chain reaction (RT-PCR), competitive reverse transcription polymerase chain reaction (competitive RT-PCR), real-time RT-PCR, RNase protection assay (RPA), northern blotting, DNA chips and the like. The expression level of mRNA in the normal control group and the expression level of mRNA of the subject in need of diagnosis as to the onset of a disease can be determined through these measurement methods, and the likelihood of the onset of a disease such as pancreatic cancer can be diagnosed or predicted through comparison between these expression levels.

When the expression level of the interleukin 10 receptor or the mRNA of the gene encoding the interleukin 10 receptor measured from the biopsy specimen isolated from a target subject is higher than that of the normal control group, the likelihood of the onset of a disease, particularly, a pancreatic disease, is determined to be high. More preferably, when the expression level measured from the biopsy specimen isolated from a target subject is at least 2 times, at least 3 times, at least 4 times, at least 5 times, at least 6 times, at least 7 times, at least 8 times or at least 9 times the expression level in the normal control group, the likelihood of the onset of a pancreatic disease is determined to be high.

In the present invention, when the expression level of the interleukin 10 receptor measured from the biopsy specimen isolated from the target subject, for example, the ratio of the number of cells expressing the interleukin 10 receptor, is 3 to 20 times, 4 to 18 times, 5 to 13 times, 7 to 11 times, or 8 to 10 times that of the normal control group, the likelihood of the onset of a pancreatic disease, particularly a pancreatic cancer, is determined to be high. Preferably, when the expression level of the interleukin 10 receptor measured from the biopsy specimen isolated from the target subject is 8 to 10 times greater than the expression level in the normal control group, the likelihood of the onset of a pancreatic disease, particularly a pancreatic cancer, is determined to be high.

In addition, in the present invention, when the expression level of mRNA of the gene encoding the interleukin 10 receptor measured from the biopsy specimen isolated from the target subject is 2 to 20 times, 2 to 15 times, 2.5 to 15 times, 2 to 10 times or 2.5 to 10 times the expression level of the normal control group, the likelihood of onset of a pancreatic disease, particularly pancreatic cancer, is determined to be high. Preferably, when the expression level of mRNA of the gene encoding the interleukin 10 receptor measured from the biopsy specimen isolated from the target subject is 2.5 to 10 times greater than the expression level in the normal control group, the likelihood of the onset of a pancreatic disease, particularly pancreatic cancer, is determined to be high.

In addition, in the present invention, when the expression level of the interleukin 22 receptor or the mRNA of the gene encoding the interleukin 22 receptor measured from the biopsy specimen isolated from a target subject is higher than the expression level of the normal control group, the likelihood of the onset of a disease, particularly, a pancreatic disease, is determined to be high. More preferably, when the expression level measured from the biopsy specimen isolated from a target subject is at least 1.5 times, at least 2 times, at least 3 times, at least 4 times, at least 5 times or at least 6 times the expression level in the normal control group, the likelihood of the onset of a pancreatic disease is determined to be high.

In addition, in the present invention, when the expression level of the interleukin 22 receptor measured from the biopsy specimen isolated from the target subject, for example, the ratio of the number of cells expressing the interleukin 22 receptor, is 2 to 10 times, 3 to 9 times, 4 to 8 times, 5 to 7 times, or 6 to 7 times that of a normal control group, the likelihood of onset of a pancreatic disease, particularly pancreatic cancer, is determined to be high. In addition, in the present invention, when the expression level of the mRNA of the gene encoding the interleukin 22 receptor measured from the biopsy specimen isolated from the target subject is 1.5 to 10 times, 1.5 to 9 times, 1.5 to 8 times, or 2 to 7 times greater than the expression level in the normal control group, the likelihood of the onset of a pancreatic disease, particularly pancreatic cancer, is determined to be high.

In the present invention, when the expression level of the interleukin 22 or mRNA of the gene encoding the interleukin 22 measured from the biopsy specimen isolated from a target subject is higher than the expression level of the normal control group, the likelihood of the onset of a disease, particularly, a pancreatic disease, is determined to be high. More preferably, when the expression level measured from the biopsy specimen isolated from a target subject is at least 2 times, at least 3 times, at least 4 times, at least 5 times or at least 6 times the expression level in the normal control group, the likelihood of the onset of a pancreatic disease is determined to be high.

In addition, in the present invention, when the expression level of the interleukin 22 measured from the biopsy specimen isolated from the target subject, for example, the ratio of the number of cells expressing the interleukin 22, is 3 to 16 times, 4 to 15 times, 4 to 13 times, 5 to 10 times, 5 to 8 times, 5 to 7 times, or 6 to 7 times that of the normal control group, the likelihood of onset of a pancreatic disease, particularly pancreatic cancer, is determined to be high.

In addition, in the present invention, when the expression level of the mRNA of the gene encoding the interleukin 22 measured from the biopsy specimen isolated from the target subject is 2 to 15 times, 2.5 to 15 times, 3 to 13 times, 3.5 to 13 times, 4 to 13 times, or 4 to 10 times that in the normal control group, the likelihood of onset of a pancreatic disease, particularly pancreatic cancer, is determined to be high.

In the present invention, when the expression level of the interleukin 29 or mRNA of the gene encoding the interleukin 29 measured from the biopsy specimen isolated from a target subject is higher than that of the normal control group, the likelihood of the onset of a disease, particularly, a pancreatic disease, is determined to be high. More preferably, when the expression level measured from the biopsy specimen isolated from a target subject is at least 2 times, at least 3 times, at least 4 times, at least 5 times or at least 6 times the expression level in the normal control group, the likelihood of the onset of a pancreatic disease is determined to be high.

In addition, in the present invention, when the expression level of the interleukin 29 measured from the biopsy specimen isolated from the target subject, for example, the ratio of the number of cells expressing the interleukin 29, is 3 to 16 times, 4 to 15 times, 4 to 13 times, 5 to 10 times, 5 to 8 times, 5 to 7 times, or 6 to 7 times that of the normal control group, the likelihood of onset of a pancreatic disease, particularly pancreatic cancer, is determined to be high.

In addition, in the present invention, when the expression level of the mRNA of the gene encoding the interleukin 29 measured from the biopsy specimen isolated from the target subject is 2 to 15 times, 2.5 to 15 times, 3 to 13 times, 3.5 to 13 times, 4 to 13 times, or 4 to 10 times that in the normal control group, the likelihood of onset of a pancreatic disease, particularly pancreatic cancer, is determined to be high.

In the present invention, when the expression level of interferon lambda receptor 1 isoform 1 or mRNA of the gene encoding the interferon lambda receptor 1 isoform 1 measured from the biopsy specimen isolated from a target subject is higher than the expression level of the normal control group, the likelihood of the onset of a disease, particularly, a pancreatic disease, is determined to be high. More preferably, when the expression level measured from the biopsy specimen isolated from a target subject is at least 1.5 times, at least 2 times, at least 3 times, at least 4 times, at least 5 times or at least 6 times the expression level in the normal control group, the likelihood of the onset of a pancreatic disease is determined to be high.

In addition, in the present invention, when the expression level of the interferon lambda receptor 1 isoform 1 measured from the biopsy specimen isolated from the target subject, for example, the ratio of the number of cells expressing the interferon lambda receptor 1 isoform 1, is 3 to 16 times, 4 to 15 times, 4 to 13 times, 5 to 10 times, 5 to 8 times, 5 to 7 times, or 6 to 7 times that of the normal control group, the likelihood of onset of a pancreatic disease, particularly pancreatic cancer, is determined to be high.

In addition, in the present invention, when the expression level of the mRNA of the gene encoding the interferon lambda receptor 1 isoform 1 measured from the biopsy specimen isolated from the target subject is 1.5 to 15 times, 2 to 15 times, 2.5 to 15 times, 3 to 13 times, 3.5 to 13 times, 4 to 13 times, or 4 to 10 times that in the normal control group, the likelihood of the onset of a pancreatic disease, particularly pancreatic cancer, is determined to be high.

In the present invention, as described above, the likelihood of onset of pancreatic cancer can be diagnosed with high accuracy by measuring the expression level of the marker of the interleukin 10 receptor, preferably the marker of the interleukin 10 receptor and the interleukin 22 receptor, more preferably, in addition to the interleukin 10 receptor and the interleukin 22 receptor, at least one marker of interleukin 22, interleukin 29 and interferon lambda receptor 1 isoform 1.

Furthermore, the method may further include subjecting a target subject to appropriate treatment such as administration of a drug for the disease (such as an anti-cancer drug for pancreatic cancer), radiation therapy or immunotherapy, when the likelihood of onset of a disease, particularly a pancreatic disease, preferably pancreatic cancer, is predicted or diagnosed to be high by measuring the expression level of at least one protein selected from the group consisting of an interleukin 10 receptor, an interleukin 22 receptor, interleukin 22, interleukin 29 and interferon lambda receptor 1 isoform 1, or the mRNA of the gene encoding the protein in the biopsy specimen isolated from the target patient.

In accordance with another aspect of the present invention, there is provided a method of screening a therapeutic drug for a disease, comprising:
(a) measuring an expression level of at least one protein selected from the group consisting of an interleukin 10 receptor, an interleukin 22 receptor, interleukin 22, interleukin 29 and interferon lambda receptor 1 isoform 1 or mRNA of the gene encoding the protein in a biopsy specimen isolated from a patient with the disease;
(b) administering a candidate drug to the patient; and
(c) measuring an expression level of at least one protein selected from the group consisting of an interleukin 10 receptor, an interleukin 22 receptor, interleukin 22, interleukin 29 and interferon lambda receptor 1 isoform 1, or the mRNA of the gene encoding the protein in the biopsy specimen isolated from the patient, after the administration of the candidate drug.

Examples of the disease that can be applied to the prediction of utility or susceptibility of a drug in the method of screening the drug according to the present invention include, but are not limited to, pancreatic diseases, autoimmune diseases, allergies, Grave's disease, Hashimoto's thyroiditis, autoimmune lymphoproliferative syndrome (ALPS), myasthenia gravis, Kawasaki disease, psoriasis, and the like.

In particular, the present invention makes it possible to more accurately predict the utility or susceptibility of a therapeutic drug for the aforementioned diseases, particularly, pancreatic diseases, specifically, pancreatic cancer or pancreatitis, more preferably, pancreatic cancer.

In the present invention, the specific types of the autoimmune diseases and allergies overlap with those described in the composition for diagnosis of the present invention, and a description thereof will be omitted below.

As used herein, the term "biopsy specimen" refers to tissue for histopathological examination application, which is collected by inserting a hollow needle or the like into an organ of the organism, rather than cutting the skin of a patient with a high possibility of developing a disease and the biopsy specimen may include a patient's tissue, cells, blood, serum, plasma, saliva, or sputum, preferably a patient's blood, serum or plasma, and more preferably a mononuclear cell or exosome isolated from the blood, serum or plasma.

The method of screening a drug according to the present invention is preferably capable of measuring the expression level of, as a biomarker, an interleukin 10 receptor or mRNA of the gene encoding the interleukin 10 receptor, from the biopsy specimen isolated from the patient in steps (a) and (c).

In addition, the method of screening a drug according to the present invention further includes measuring the expression level of an interleukin 22 receptor or mRNA of the gene encoding the interleukin 22 receptor, in addition to the expression level of the interleukin 10 receptor or mRNA of the gene encoding the interleukin 10 receptor, from the biopsy specimen isolated from the patient in steps (a) and (c), thereby further improving the accuracy of the prediction of susceptibility of a therapeutic drug.

In addition, the method of screening a drug according to the present invention further includes measuring the expression level of at least one protein selected from the group consisting of interleukin 22, interleukin 29 and interferon lambda receptor 1 isoform 1, or mRNA of a gene encoding the protein, in addition to the expression level of the interleukin 10 receptor, interleukin 22 receptor or mRNAs of the genes encoding these receptors, from the biopsy specimen isolated from the patient in steps (a) and (c), thereby further improving the accuracy of the prediction of susceptibility of the therapeutic drug for the disease.

The agent for measuring the expression level of the interleukin 10 receptor, the interleukin 22 receptor, interleukin 22, the interleukin 29, or the interferon lambda receptor 1 isoform 1 is not particularly limited, but preferably includes at least one selected from the group consisting of antibodies, oligopeptides, ligands, PNAs (peptide nucleic acids) and aptamers that specifically bind to each of the interleukin 10 receptor, the interleukin 22 receptor, the interleukin 22, the interleukin 29 or the interferon lambda receptor 1 isoform 1.

Preferably, in the present invention, the expression level of the interleukin 10 receptor, interleukin 22 receptor, interleukin 22, interleukin 29, or interferon lambda receptor 1 isoform 1 can be measured and compared using antibodies specifically binding to the interleukin 10 receptor, the interleukin 22 receptor, the interleukin 22, interleukin 29, or the interferon lambda receptor 1 isoform 1. This can be carried out using a method of detecting a antigen-antibody complex which is formed by the antibody with the corresponding protein in the biological sample.

As used herein, the term "antigen-antibody complex" refers to a combination of a protein antigen for detecting the presence or absence of the corresponding gene in the biological sample with an antibody recognizing the protein antigen. The detection of the antigen-antibody complex can be carried out using any of methods well-known in the art, for example, spectroscopic, photochemical, biochemical, immunochemical, electrical, absorption spectrometric, chemical and other methods.

Meanwhile, in the present invention, the methods for measurement of expression levels of proteins or comparative analysis thereof include, but are not limited to, protein chip analysis, immunoassay, ligand-binding assay, MALDI-TOF (matrix assisted laser desorption/ionization time of flight mass spectrometry) analysis, radiation immunoassay, radiation immunodiffusion, SELDI-TOF (surface enhanced laser desorption/ionization time of flight mass spectrometry) analysis, radiation immunoassay, radiation immunodiffusion, ouchterlony immunodiffusion, rocket immunoelectrophoresis, tissue immunostaining, complement fixation analysis, 2D electrophoresis analysis, liquid chromatography-mass spectrometry (LC-MS), liquid chromatography-mass spectrometry/mass spectrometry (LC-MS/MS), western blotting, enzyme-linked immunosorbent assay (ELISA) and the like.

In addition, in the present invention, the agent for measuring the expression level of the mRNA of the gene encoding each of the interleukin 10 receptor, the interleukin 22 receptor, the interleukin 22, the interleukin 29 or the interferon lambda receptor 1 isoform 1 may include at least one selected from the group consisting of primers, probes and antisense nucleotides that specifically bind to the mRNA of the gene encoding the protein of each of the interleukin 10 receptor, the interleukin 22 receptor, the interleukin 22, the interleukin 29 or the interferon lambda receptor 1 isoform 1. Since the information of the interleukin 10 receptor, the interleukin 22 receptor, the interleukin 22, the interleukin 29 and the interferon lambda receptor 1 isoform 1 according to the present invention is known, those skilled in the art can easily design primers, probes, or antisense nucleotides specifically binding to the mRNA of the gene encoding the protein based on the information.

In the present invention, the measurement and comparison of expression levels of the mRNA of the gene encoding the interleukin 10 receptor, the interleukin 22 receptor, the interleukin 22, the interleukin 29 or the interferon lambda receptor 1 isoform 1 may be carried out using a method including, but not limited to, reverse transcription polymerase chain reaction (RT-PCR), competitive reverse transcription polymerase chain reaction (competitive RT-PCR), real-time RT-PCR, RNase protection assay (RPA), northern blotting, DNA chips and the like. The expression level of mRNA in the normal control group and the expression level of mRNA of the subject in need of diagnosis as to the onset of a disease can be determined through these measurement methods, and the likelihood of the onset of a disease such as pancreatic cancer can be diagnosed or predicted through comparison between these expression levels.

The method according to the present invention may further include selecting the candidate drug as a therapeutic drug suitable for the treatment of a disease, when the expression level of at least one protein selected from the group consisting of an interleukin 10 receptor, an interleukin 22 receptor, interleukin 22, interleukin 29 and interferon lambda receptor 1 isoform 1, or the mRNA of the gene encoding the protein measured in step (c) is decreased than the expression level of the same biomarker measured in step (a).

### [Advantageous effects]

The present invention makes it possible to accurately predict and diagnose a variety of diseases, such as pancreatic diseases, autoimmune diseases, allergies, Grave's disease, Hashimoto's thyroiditis, psoriasis, Kawasaki disease and autoimmune lymphoproliferative syndrome (ALPS), particularly, pancreatic cancer, by measuring the expression levels of proteins of interleukin 10 receptor, interleukin 22 receptor, interleukin 22, interleukin 29, and/or interferon lambda receptor 1 isoform 1.

In addition, the present invention makes it possible to predict or diagnose the onset of diseases by measuring the expression level of the biomarker from the mononuclear cells or exosomes present in the blood, serum or plasma isolated from the target subject, thereby more rapidly and simply, yet more accurately diagnosing a disease such as pancreatic cancer in a non-invasive manner compared to conventional cases.

### [Description of Drawings]

Reference will now be made in detail to the preferred embodiments of the present invention, examples of which are illustrated in the accompanying drawings. Wherever possible, the same reference numbers will be used throughout the drawings to refer to the same or like parts.
FIG. 1 shows the proportion (%) of the number of mononuclear cells expressing IL-22 relative to the total number of mononuclear cells in blood collected from the normal control group, pancreatic cancer patients, pancreatitis patients, lung cancer patients and colorectal cancer patients using FACS analysis in Example 1.
FIG. 2 shows the proportion (%) of the number of mononuclear cells expressing IL-22R relative to the total number of mononuclear cells in blood collected from the normal control group, pancreatic cancer patients, pancreatitis patients, lung cancer patients and colorectal cancer patients using FACS analysis in Example 1.
FIG. 3 shows the proportion (%) of the number of mononuclear cells expressing IL-10R relative to the total number of mononuclear cells in blood collected from the normal control group, pancreatic cancer patients, pancreatitis patients, lung cancer patients and colorectal cancer patients using FACS analysis in Example 1.
FIG. 4 is a graph showing comparison of mRNA expression levels of IL-10RB in mononuclear cells in the blood collected from the normal control group, pancreatic cancer patients, cholangiocarcinoma patients and gallbladder cancer patients in Example 2.
FIG. 5 is a graph showing comparison of mRNA expression levels of IL-10RA in mononuclear cells in the blood collected from the normal control group, pancreatic cancer patients, cholangiocarcinoma patients and gallbladder cancer patients in Example 2.
FIG. 6 is a graph showing comparison of mRNA expression levels of IL-22 in mononuclear cells in the blood collected from the normal control group, pancreatic cancer patients, cholangiocarcinoma patients and gallbladder cancer patients in Example 3.
FIG. 7 is a graph showing comparison of mRNA expression levels of IL-29 in mononuclear cells in the blood collected from the normal control group, pancreatic cancer patients, cholangiocarcinoma patients and gallbladder cancer patients in Example 3.
FIG. 8 is a graph showing comparison of mRNA expression levels of IFNLR1 isoform 1 in mononuclear cells in the blood collected from the normal control group, pancreatic cancer patients, cholangiocarcinoma patients and gallbladder cancer patients in Example 4.
FIG. 9 is a graph showing comparison of mRNA expression levels of IFNLR1 isoform 3 in mononuclear cells in the blood collected from the normal control group, pancreatic cancer patients, cholangiocarcinoma patients and gallbladder cancer patients in Example 4.
FIG. 10 shows the proportion (%) of the number of mononuclear cells expressing IL-22 relative to the total number of mononuclear cells in blood collected from pancreatic cancer patients before and after surgery in Example 5.
FIG. 11 shows the proportion (%) of the number of mononuclear cells expressing IL-22R relative to the total number of mononuclear cells in blood collected from pancreatic cancer patients before and after surgery in Example 5.
FIG. 12 shows the proportion (%) of the number of mononuclear cells expressing IL-10R relative to the total number of mononuclear cells in blood collected from pancreatic cancer patients before and after surgery in Example 5.
FIG. 13 is a graph showing the results of analysis through paired T test after measuring the proportion of the number of mononuclear cells expressing IL-10R relative to the total number of mononuclear cells in blood collected from pancreatic cancer patients before surgery and 3 months after surgery in Example 6.
FIG. 14 is a graph showing comparison of mRNA expression levels of IL-10RB in exosomes collected from the normal control group, pancreatic cancer patients, cholangiocarcinoma patients and gallbladder cancer patients in Example 7.
FIG. 15 is a graph showing comparison of mRNA expression levels of IL-22RA in exosomes collected from the normal control group, pancreatic cancer patients, cholangiocarcinoma patients and gallbladder cancer patients in Example 7.
FIG. 16 is a graph showing comparison of mRNA expression levels of IFNLR1 isoform 1 in exosomes collected from the normal control group, pancreatic cancer patients, cholangiocarcinoma patients and gallbladder cancer patients in Example 7.

### [Best Mode for Invention]

According to an embodiment of the present invention, the present invention is directed to a composition for diagnosing a disease containing an agent for measuring an expression level of at least one protein selected from the group consisting of an interleukin 10 receptor (IL-10R), an interleukin 22 receptor (IL-22R), interleukin 22 (IL-22), interleukin 29 (IL-29), and interferon lambda receptor 1 isoform 1 (IFNLR1 isoform 1), or mRNA of a gene encoding the protein.

According to another embodiment of the present invention, the present invention is directed to a kit for diagnosing a disease containing the composition for diagnosis according to the present invention.

According to yet another embodiment of the present invention, the present invention is directed to a method of providing information for diagnosis of a disease including measuring an expression level of at least one protein selected from the group consisting of an interleukin 10 receptor (IL-10R), an interleukin 22 receptor (IL-22R), interleukin 22 (IL-22), interleukin 29 (IL-29), or interferon lambda receptor 1 isoform 1 (IFNLR1 isoform 1), or mRNA of a gene encoding the protein in a biopsy specimen isolated from a target subject.

According to yet another embodiment of the present invention, the present invention is directed to a method of screening a therapeutic drug for a disease comprising (a) measuring an expression level of at least one protein selected from the group consisting of an interleukin 10 receptor, an interleukin 22 receptor, interleukin 22, interleukin 29 and interferon lambda receptor 1 isoform 1 or mRNA of the gene encoding the protein in a biopsy specimen isolated from a patient with the disease, (b) administering a candidate drug to the patient, and (c) measuring the expression level of at least one protein selected from the group consisting of an interleukin 10 receptor, an interleukin 22 receptor, interleukin 22, interleukin 29 and interferon lambda receptor 1 isoform 1, or the mRNA of the gene encoding the protein in the biopsy specimen isolated from the patient, after the administration of the candidate drug.

### [Mode for Invention]

Hereinafter, the present invention will be described in more detail with reference to the following examples. However, the following examples are provided only for illustration of the present invention and should not be construed as limiting the scope of the present invention.

### [Preparation Example 1]

Histopaq 1077 was purchased from Sigma-Aldrich Corporation, and Brefeldin A was purchased from BioLegend Inc. As antibodies, Alexa 647 anti-IL-22 and PE anti-mouse IL-10R were purchased from BioLegend Inc., and PerCP mouse IL-22R was purchased from R & D. PBS (phosphate buffered solution) containing 0.5% bovine serum albumin (BSA) was used as a FACS buffer. A cell fixation buffer and a perm/wash buffer were also purchased from BioLegend Inc.

### [Example 1]

### 1. Isolation of peripheral blood mononuclear cells (PBMCs)

Blood samples were collected from 3 pancreatic cancer patients, 3 pancreatitis patients, 3 normal control group subjects, 3 lung cancer patients and 3 colorectal cancer patients and stored at room temperature. Since the separation of mononuclear cells is difficult when blood viscosity is high, blood samples were prepared through 1:1 dilution in 1X PBS. Ficoll (Histopaque 1077, Sigma) was prepared in the same volume as blood in a 15 ml conical tube. Blood samples were added onto Ficoll such that the blood samples were not mixed. The resulting blood samples were centrifuged at 400 g for 30 minutes with minimum acceleration and deceleration. During this process, a whitecell (mononuclear cell) layer was separately collected, and PBS was added thereto to conduct washing with centrifugation at 400 g for 3 minutes. The washed cells were collected, resuspended in 50 µl FACS buffer, and then cultured on ice for 1 hour with a PE anti-mouse IL-10R antibody and a PerCP mouse IL-22R antibody. The cells were then washed twice with FACS buffer and resuspended in 500 µl FACS buffer.

### 2. Treatment with Brefeldin A

The collected cells were counted, seeded at 1×10⁶ cells/ml in RPMI 1640 medium containing 2% penicillin and streptomycin, treated with 1X Brefeldin A and cultured for 6 hours.

### 3. Fixing, perming and staining

The cultured cells were collected, washed twice with FACS buffer (0.5% BSA in PBS), and fixed with 500 µl of fixed buffer on ice for 30 minutes. Then, the cells were washed twice with FACS buffer, resuspended in 1 ml of perm buffer and then centrifuged twice for 20 minutes. Then, the cells were collected again, resuspended in 50 µl of perm buffer and then cultured on ice for 1 hour with Alexa 647 anti-IL-22 antibody. The cells were washed twice with FACS and resuspended in 500 µl of FACS buffer.

### 4. FACS analysis

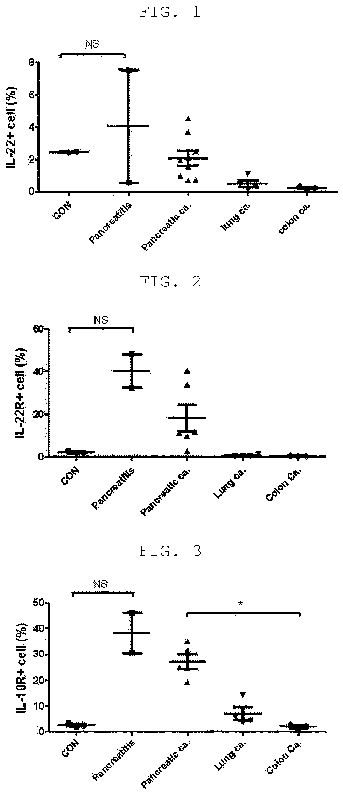

The number of cells expressing IL-10R, IL-22R or IL-22 relative to the total number of mononuclear cells was expressed in % using a FACSCalibur (BD Biosciences, San Jose, CA). The results are shown in the following Table 1 and FIGS. 1 to 3. In the following Table 1, data are expressed as mean ± SD, and statistical analysis was performed using the Kruskal-Wallis test using Dunn's post hoc analysis.

**[Table 1]**

| Item | Mean ± SD (%) | | | | |
|---|---|---|---|---|---|
| | Normal control group | Pancreatitis | Pancreatic cancer | Lung cancer | Colorectal cancer |
| IL-22 | 2.44±0.02 | 4.06±3.49 | 2.25±1.31 | 0.51±0.43 | 0.21±0.01 |
| IL-22R | 2.16±0.49 | 40.30±8.00 | 14.20±14.88 | 0.60±0.55 | 0.37±0.13 |
| IL-10R | 2.59±0.75 | 38.40±7.80 | 23.20±8.65 | 7.13±4.93 | 2.01±0.89 |

As shown in Table 1 and FIGS. 1 to 3, the expression levels of IL-22 in pancreatitis patients and pancreatic cancer patients correspond to about 1.7 times and 0.9 times relative to the expression level of IL-22 in the normal control group respectively, but the expression level of IL-10R, to which IL-22 binds, was about 14.8 times increased in the pancreatitis patients than in the normal control group, and was about 9.0 times increased in the pancreatic cancer patients than in the normal control group. However, the expression level of IL-10R was about 2.75 times increased in lung cancer patients than in the normal control group, and the expression level of IL-10R was about 0.78 times decreased in the colorectal cancer patients than in the normal control group.

In addition, it was found that, regarding the expression level of IL-22R, the pancreatitis patients had an increase of about 18.66 times compared to the normal control group, and the pancreatic cancer patients had an increase of about 6.57 times compared to the normal control group. However, the lung cancer patients had a decrease of about 0.28 times compared to the normal control group and the colorectal cancer patients had a notable decrease of about 0.17 times compared to the normal control group.

Further, regarding the expression level of IL-22R relative to IL-22, the expression level of IL-22R relative to the expression level of IL-22 in pancreatitis patients was about 9.93 times, and the expression level of IL-22R relative to the expression level of IL-22 in pancreatic cancer was about 6.31 times. This indicates that, although IL-22 binds to IL-22R, the expression level of IL-22R was significantly increased compared to IL-22. However, the expression level of IL-22R relative to the expression level of IL-22 in lung cancer patients was about 1.18 times, and the expression level of IL-22R relative to the expression level of IL-22 in colorectal cancer patients was about 1.76 times. This indicates that the expression level of IL-22 is similar to that of IL-22R.

These results demonstrate that the expression level of IL-10R in pancreatitis or pancreatic cancer patients is clearly differentiated from that of the normal control group or patients of other diseases, and pancreatitis and pancreatic cancer also have distinct expression behaviors.

Therefore, with the method according to the present invention, pancreatitis and pancreatic cancer can be detected and diagnosed with high accuracy, and the diagnosis can be carried out while distinguishing between pancreatitis and pancreatic cancer.

### [Example 2]

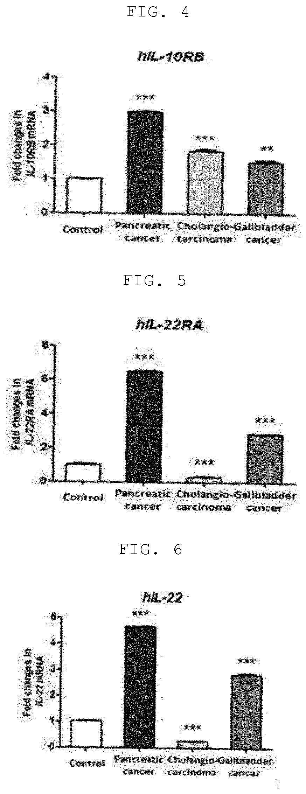

Blood samples were collected from 4 normal control group subjects, 4 pancreatic cancer patients, 2 cholangiocarcinoma patients and 2 gallbladder cancer patients, and peripheral blood mononuclear cells (PBMCs) were separated from the blood samples in the same manner as in Example 1, and the ratio of mRNA expression levels of IL-10R beta subunits (IL-10RB) and IL-22R alpha subunits (IL-22RA) in the PBMCs compared to the normal control group was measured, and the results are shown in FIGS. 4 and 5.

As shown in FIGS. 4 and 5, IL-10RB mRNA expression level increased about 3 times and IL-22RA mRNA expression level increased about 6 times, respectively, in PBMCs isolated from pancreatic cancer patients compared to the normal control group, but the expression level of IL-10RB mRNA in cholangiocarcinoma and gallbladder cancer patients increased about 1 to 2 times, and the expression level of IL-22RA mRNA decreased in cholangiocarcinoma patients.

These results indicate that the mRNA expression levels of IL-10RB and IL-22RA detected in PBMCs were significantly increased in pancreatic cancer compared to other types of cancer.

### [Example 3]

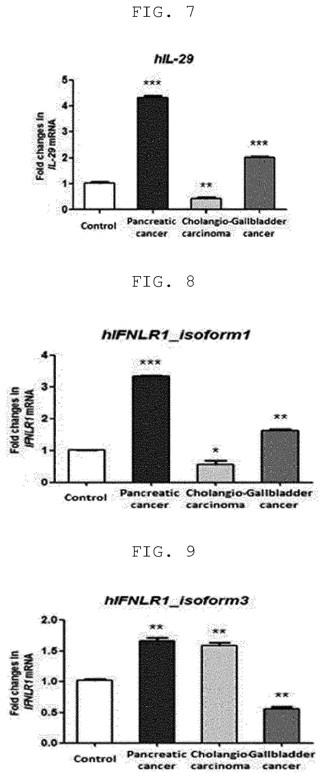

Blood samples were collected from 4 normal control group subjects, 4 pancreatic cancer patients, 2 cholangiocarcinoma patients and 2 gallbladder cancer patients, and peripheral blood mononuclear cells (PBMCs) were separated from the blood samples in the same manner as in Example 1, and the ratio of mRNA expression levels of IL-22 and IL-29 in the PBMCs compared to the normal control group was measured, and the results are shown in FIGS. 6 and 7.

As shown in FIGS. 6 and 7, the expression levels of IL-22 mRNA and IL-29 mRNA were increased about 4 times or more in PBMCs isolated from pancreatic cancer patients compared to the normal control group, but the expression levels of the two markers in cholangiocarcinoma patients were decreased, and the expression levels thereof in gallbladder cancer patients were increased only about 2 to 3 times.

These results indicate that the mRNA expression levels of IL-22 and IL-29 detected in PBMCs were significantly increased in pancreatic cancer compared to other types of cancer.

### [Example 4]

Blood samples were collected from 4 normal control group subjects, 4 pancreatic cancer patients, 2 cholangiocarcinoma patients and 2 gallbladder cancer patients, peripheral blood mononuclear cells (PBMCs) were then separated from the blood samples in the same manner as in Example 1, and the ratio of mRNA expression levels of interferon receptor 1 isoform 1 (IFNLR1 isoform 1) and interferon receptor 1 isoform 3 (IFNLR1 isoform 3), which bind to IL-29, in the PBMCs compared to the normal control group was measured, and the results are shown in FIGS. 8 and 9.

As shown in FIGS. 8 and 9, the mRNA expression level of interferon receptor 1 isoform 1, which binds to IL-29, increased about 3 times or more in PBMCs isolated from pancreatic cancer patients compared to the normal control group, but increased about 1 to 2 times in cholangiocarcinoma and gallbladder cancer patients.

These results indicate that the mRNA expression level of interferon receptor 1 isoform 1 detected in PBMCs was significantly increased in pancreatic cancer compared to other types of cancer.

### [Example 5]

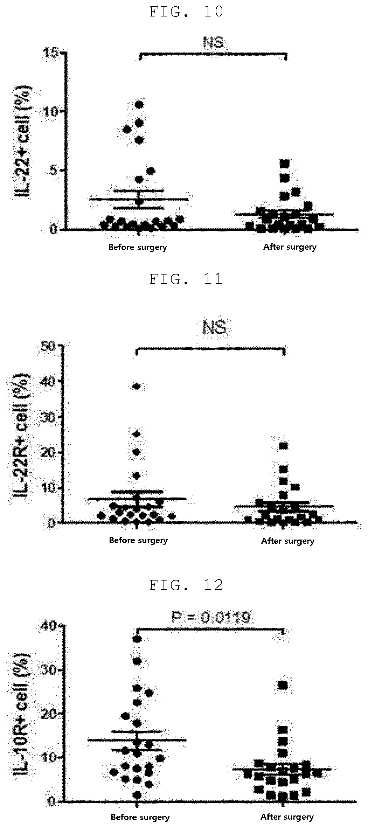

Blood samples were collected from pancreatic cancer patients who underwent pancreatic cancer resection (pancreatotomy) before and after the surgery, PBMCs were then isolated from the blood samples in the same manner as in Example 1, and the number of cells expressing IL-10R, IL-22R or IL-22 relative to the total number of mononuclear cells was expressed in % using a FACSCalibur (BD Biosciences, San Jose, CA). The results are shown in the following Table 2 and FIGS. 10 to 12.

**[TABLE 2]**

| Item | Mean ± SD (%) | |
|---|---|---|
| | Before surgery | After surgery |
| IL-22 | 2.56±3.45 | 1.30±1.53 |
| IL-22R | 6.98±9.75 | 4.65±5.78 |
| IL-10R | 13.89±9.74 | 7.41±5.85 |

As can be seen from Table 2 and FIGS. 10 to 12, when the total mass of pancreatic cancer tissue is reduced due to pancreatotomy of pancreatic cancer patients, the expression levels of IL-10R, IL-22 and IL-22R in mononuclear cells are remarkably decreased, and in particular, the expression level of IL-10R is significantly decreased.

### [Example 6]

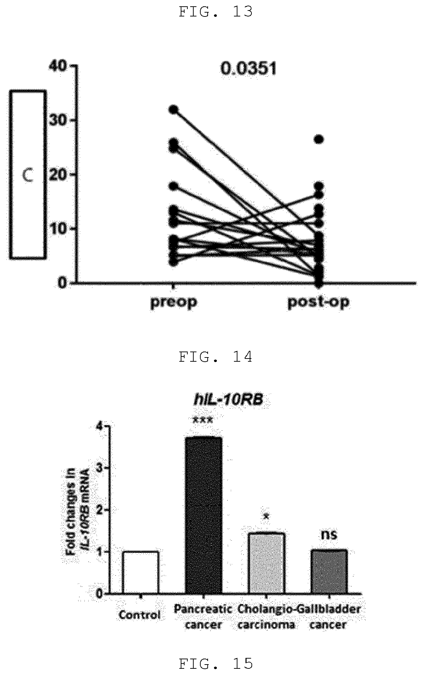

Blood samples were collected from pancreatic cancer patients who underwent pancreatic cancer resection (pancreatotomy) before surgery and 3 months after surgery, PBMCs were then isolated from the blood samples in the same manner as in Example 1, and the number of cells expressing IL-10R (marker C) relative to the total number of mononuclear cells was measured using a FACSCalibur (BD Biosciences, San Jose, CA) and then analyzed using a paired T test. The results are shown in FIG. 13.

As shown in FIG. 13, the proportion of mononuclear cells expressing IL-10R was significantly decreased after the cancer tissue was removed from pancreatic cancer patients using pancreatotomy compared to before surgery.

### [Example 7]

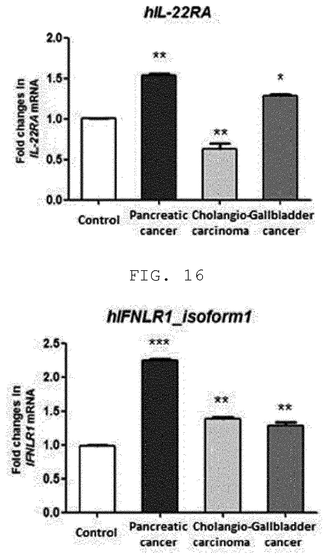

Blood samples were collected from 4 normal control group subjects, 4 pancreatic cancer patients, 2 cholangiocarcinoma patients and 2 gallbladder cancer patients, exosomes circulating in the plasma were separated from the blood samples, and the ratio of mRNA expression levels of IL-10R beta subunit (IL-10RB), IL-22R alpha 1 (IL-22RA) and interferon receptor 1 isoform 1 (IFNLR1 isoform 1) in the exosomes compared to the normal control group was measured, and the results are shown in FIGS. 14 to 16.

As can be seen from FIGS. 14 to 16, the expression levels of IL-10RB mRNA, IL-22RA mRNA and interferon receptor 1 isoform 1 mRNA were about 4 times, about 1.5 times and about 2.5 times increased, respectively, in the exosomes isolated from pancreatic cancer patients than in the normal control group. However, in the exosomes isolated from cholangiocarcinoma patients, the expression level of IL-10RB mRNA was about 1.5 times increased than that of the normal control group, and the expression level of interferon receptor 1 isoform 1 mRNA was only about 1.5 times increased than that of the normal control group, particularly, the expression level of IL-22RA mRNA was decreased compared to the normal control group. In the case of gallbladder cancer patients, the expression level of IL-10RB mRNA was equivalent to that of the normal control group in the exosomes isolated therefrom, and the mRNA expression levels of IL-22RA and interferon receptor 1 isoform 1 were about 1.5 times higher at most compared to the normal control group.

The results showed that the expression levels of IL-10RB, IL-22RA and interferon receptor 1 isoform 1 mRNA expressed in exosomes isolated from plasma in pancreatic cancer patients were significantly increased compared to the normal control group and other cancer patients.

### [Industrial Applicability]

The present invention relates to a composition for diagnosis of a variety of diseases such as pancreatic cancer, a kit for diagnosis containing the same, and a method of providing information for diagnosis using the composition.

### [Sequence Listing Free Text]

SEQ ID NO: 1 (IL-10RB): 
maawslgswlggcllvsalgmvpppenvrmnsvnfknilqwespafakgnl

tftaqylsyrifqdkcmnttltecdfsslskygdhtlrvraefadehsdwvnitfcpvddtiigppgmqvevladslhmrfl

apkieneyetwtmknvynswtynvqywkngtdekfqitpqydfevlrnlepwttycvqvrgflpdrnkagewsepv

ceqtthdetvpswmvavilmasvfmvclallgcfallwcvykktkyafsprnslpqhlkeflghphhntllffsfplsden

dvfdklsviaedsesgkqnpgdscslgtppgqgpqs
SEQ ID NO: 2 (IL-22RA): 
mrtllltiltvgslaahapedpsdllqhvkfqssnfeniltwdsgpegtpd

tvysieyktygerdwvakkgcqritrkscnltvetgnltelyyarvtavsaggrsatkmtdrfsslqhttlkppdvtciskvrs

iqmivhptptpiragdghrltledifhdlfyhlelqvnrtyqmhlggkqreyeffgltpdteflgtimicvptwakesapy

mcrvktlpdrtwtysfsgaflfsmgflvavlcylsyryvtkppappnslnvqrvltfqplrfiqehvlipvfdlsgpsslaqp
 
vqysqirvsgprepagapqrhslseitylgqpdisilqpsnvpppqilsplsyapnaapevgppsyapqvtpeaqfpfya

pqaiskvqpssyapqatpdswppsygvcmegsgkdsptgtlsspkhlrpkgqlqkeppagscmlgglslqevtslame

esqeakslhqplgictdrtsdpnvlhsgeegtpqylkgqlpllssvqieghpmslplqppsrpcspsdqgpspwglleslv

cpkdeakspapetsdleqpteldslfrglaltvqwes
SEQ ID NO: 3 (IL-22): 
maalqksvssflmgtlatsclllllallvqggaaapisshcrldksnfqqpyitnrtfmlakeasladnntdvrligeklfhgvs
msercylmkqvlnftleevlfpqsdrfqpymqevvpflarlsnrlstchiegddlhiqrnvqklkdtvkklgesgeikaig
eldllfmslrnaci
SEQ ID NO: 4 (IL-29): 
maaawtvvlvtlvlglavagpvptskptttgkgchigrfkslspqelasfkkardaleeslklknwscsspvfpgndlrllq

vrerpvaleaelaltlkvleaaagpaledvldqplhtlhhilsqlqaciqpqptagprprgrlhhwlhrlqeapkke

sagcleasvt fnlfrllltrdlkyvadgnlclrtsthpest
SEQ ID NO: 5 (IFNLR1 isoform 1) :

magperwgplllcllqaapgrprlappqnvtllsqnfsvyltwlpglgnpqdvtyfvayqssptrrrwreveecagtkellc
smmclkkqdlynkfkgrvrtvspsskspwveseyldylfevepappvlvltqteeilsanatyqlppcmppldlkyeva
fwkegagnktlfpvtphgqpvqitlqpaasehhclsartiytfsvpkyskfskptcfllevpeanwaflvlpsllilllviaag
gviwktlmgnpwfqrakmpraldfsghthpvatfqpsrpesvndlflcpqkeltrgvrptprvrapatqqtrwkkdlaed
eeeedeedtedgvsfqpyieppsflgqehqapghseaggvdsgrpraplvpsegssawdssdrswastvdsswdrags
sgylaekgpgqgpggdghqeslpppefskdsgfleelpednlsswatwgtlppepnlvpggppvslqtltfcwesspee
eeeareseiedsdagswgaestqrtedrgrtlghymar

The following numbered paragraphs (paras.) contain further statements of various aspects of the present invention:
[Para 1] A composition for diagnosing a pancreatic disease comprising an agent for measuring an expression level of at least one protein selected from the group consisting of an interleukin 10 receptor (IL-10R), an interleukin 22 receptor (IL-22R), interleukin 22 (IL-22), interleukin 29 (IL-29), and interferon lambda receptor 1 isoform 1 (IFNLR1 isoform 1), or mRNA of a gene encoding the protein.
[Para 2] The composition according to para 1, wherein the composition is to a mononuclear cell isolated from a target subject.
[Para 3] The composition according to para 1, wherein the composition comprises an agent for measuring an expression level of the interleukin 10 receptor or mRNA of the gene encoding the interleukin 10 receptor.
[Para 4] The composition according to para 3, wherein the composition further comprises an agent for measuring an expression level of the interleukin 22 receptor or mRNA of the gene encoding the interleukin 22 receptor.
[Para 5] The composition according to para 3, wherein the composition further comprises an agent for measuring an expression level of at least one protein selected from the group consisting of interleukin 22 (IL-22), interleukin 29 (IL-29), and interferon lambda receptor 1 isoform 1 (IFNLR1 isoform 1), or mRNA of the gene encoding the protein.
[Para 6] The composition according to para 1, wherein the agent for measuring the expression level of the protein comprises at least one selected from the group consisting of antibodies, oligopeptides, ligands, PNAs (peptide nucleic acids) and aptamers that specifically bind to the protein.
[Para 7] The composition according to para 1, wherein the agent for measuring the expression level of the mRNA comprises at least one selected from the group consisting of primers, probes and antisense nucleotides that specifically bind to the mRNA.
[Para 8] The composition according to para 1, wherein the pancreatic disease is a pancreatic cancer.
[Para 9] A kit for diagnosing a pancreatic disease comprising the composition for diagnosis according to any one of paras 1 to 8.
[Para 10] The kit according to para 9, wherein the kit is an RT-PCR kit, a DNA chip kit, an ELISA kit, a protein chip kit, a rapid kit, or a multiple reaction-monitoring (MRM) kit.
[Para 11] A method of providing information for diagnosis of a pancreatic disease comprising measuring an expression level of at least one protein selected from the group consisting of an interleukin 10 receptor (IL-10R), an interleukin 22 receptor (IL-22R), interleukin 22 (IL-22), interleukin 29 (IL-29), or interferon lambda receptor 1 isoform 1 (IFNLR1 isoform 1), or mRNA of a gene encoding the protein in a biopsy specimen isolated from a target subject.
[Para 12] The method according to para 11, wherein the biopsy specimen is at least one selected from the group consisting of blood, serum and plasma isolated from the subject.
[Para 13] The method according to para 12, wherein the biopsy specimen comprises at least one of a mononuclear cell or exosome isolated from the subject.
[Para 14] The method according to para 11, comprising measuring an expression level of the interleukin 10 receptor, or mRNA of a gene encoding the interleukin 10 receptor from the biopsy specimen.
[Para 15] The method according to para 14, further comprising measuring an expression level of the interleukin 22 receptor, or mRNA of a gene encoding the interleukin 22 receptor from the biopsy specimen.
[Para 16] The method according to para 14, further comprising measuring an expression level of at least one protein selected from the group consisting of interleukin 22, interleukin 29 and interferon lambda receptor 1 isoform 1, or mRNA of a gene encoding the protein from the biopsy specimen.
[Para 17] The method according to para 11, wherein likelihood of the onset of the pancreatic disease is determined to be high, when the expression level of the protein or the mRNA measured from the biopsy specimen isolated from the target subject is higher than an expression level of a normal control group.
[Para 18] The method according to para 14, wherein likelihood of the onset of the pancreatic disease is determined to be high, when the expression level of the interleukin 10 receptor or the mRNA of the gene encoding the interleukin 10 receptor measured from the biopsy specimen isolated from the target subject is 2 times or more the expression level of the normal control group.
[Para 19] The method according to para 15, wherein likelihood of the onset of the pancreatic disease is determined to be high, when the expression level of the interleukin 22 receptor or the mRNA of the gene encoding the interleukin 22 receptor measured from the biopsy specimen isolated from the target subject is higher than an expression level of a normal control group.
[Para 20] The method according to para 19, wherein likelihood of the onset of the pancreatic disease is determined to be high, when the expression level of the interleukin 22 receptor or the mRNA of the gene encoding the interleukin 22 receptor measured from the biopsy specimen isolated from the target subject is 1.5 times or more the expression level of the normal control group.
[Para 21] The method according to para 11, wherein the pancreatic disease is a pancreatic cancer.
[Para 22] A method of screening a therapeutic drug for a disease comprising:
   (a) measuring an expression level of at least one protein selected from the group consisting of an interleukin 10 receptor, an interleukin 22 receptor, interleukin 22, interleukin 29 and interferon lambda receptor 1 isoform 1 or mRNA of a gene encoding the protein in a biopsy specimen isolated from a patient with the disease;
   (b) administering a candidate drug to the patient; and
   (c) measuring the expression level of at least one protein selected from the group consisting of an interleukin 10 receptor, an interleukin 22 receptor, interleukin 22, interleukin 29 and interferon lambda receptor 1 isoform 1, or the mRNA of the gene encoding the protein in the biopsy specimen isolated from the patient, after the administration of the candidate drug.

## Claims

1. A use of a composition for the diagnosis of a pancreatic disease comprising an agent for measuring an expression level of an interleukin 29 (IL-29) protein, or mRNA of a gene encoding the protein.

2. The use according to claim 1, wherein the expression level is measured in a biological sample of mononuclear cells.

3. The use according to claim 1, wherein the composition further comprises an agent for measuring an expression level of at least one protein selected from the group consisting of interleukin 22 receptor (IL-22R), interleukin 22 (IL-22), and interferon lambda receptor 1 isoform 1 (IFNLR1 isoform 1), or mRNA of the gene encoding the protein.

4. The use according to claim 1, wherein the agent for measuring the expression level of the protein comprises at least one selected from the group consisting of antibodies, oligopeptides, ligands, PNAs (peptide nucleic acids) and aptamers that specifically bind to the protein.

5. The use according to claim 1, wherein the agent for measuring the expression level of the mRNA comprises at least one selected from the group consisting of primers, probes and antisense nucleotides that specifically bind to the mRNA.

6. The use according to claim 1, wherein the pancreatic disease is a pancreatic cancer.

7. A use of a kit for the diagnosis of a pancreatic disease comprising an agent for measuring an expression level of an interleukin 29 (IL-29) protein, or mRNA of a gene encoding the protein.

8. The use according to claim 7, wherein the kit is an RT-PCR kit, a DNA chip kit, an ELISA kit, a protein chip kit, a rapid kit, or a multiple reaction-monitoring (MRM) kit.

9. A method of screening a therapeutic drug for a pancreatic disease comprising:
(a) measuring an expression level of an interleukin 29 (IL-29) protein, or mRNA of a gene encoding the protein in a biopsy specimen isolated from a patient with the disease;
(b) administering a candidate drug to the patient; and
(c) measuring the expression level of an interleukin 29 (IL-29) protein, or the mRNA of the gene encoding the protein in the biopsy specimen isolated from the patient, after the administration of the candidate drug.
